(19) Europäisches Patentamt / European Patent Office / Office européen des brevets

(11) EP 2 866 563 B1

(12) **EUROPEAN PATENT SPECIFICATION**

(45) Date of publication and mention of the grant of the patent:
**31.07.2019 Bulletin 2019/31**

(21) Application number: **13810805.5**

(22) Date of filing: **12.06.2013**

(51) Int Cl.:
***A01N 47/40*** *(2006.01)* ***A01N 47/34*** *(2006.01)*
***C07D 213/89*** *(2006.01)* ***A01N 51/00*** *(2006.01)*
***A01P 7/04*** *(2006.01)*

(86) International application number:
**PCT/US2013/045333**

(87) International publication number:
**WO 2014/004086 (03.01.2014 Gazette 2014/01)**

(54) **INSECTICIDAL N-SUBSTITUTED SULFILIMINE AND SULFOXIMINE PYRIDINE N-OXIDES**

INSEKTIZIDE N-SUBSTITUIERTE SULFILIMIN- UND SULFOXIMINPYRIDIN-N-OXIDE

N-OXYDES DE PYRIDINE SULFILIMINE ET SULFOXIMINE N-SUBSTITUÉS INSECTICIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2012 US 201261666814 P**

(43) Date of publication of application:
**06.05.2015 Bulletin 2015/19**

(73) Proprietor: **Dow AgroSciences LLC**
**Indianapolis IN 46268-1054 (US)**

(72) Inventors:
- **BLAND, Douglas, C.**
  **Midland, MI 48642 (US)**
- **ROSS, Ronald Jr.**
  **Zionsville, IN 46077 (US)**
- **JOHNSON, Peter, L.**
  **Indianapolis, IN 46256 (US)**
- **JOHNSON, Timothy, C.**
  **Indianapolis, IN 46260 (US)**

(74) Representative: **HGF Limited**
**4th Floor**
**Merchant Exchange**
**17-19 Whitworth Street West**
**Manchester M1 5WG (GB)**

(56) References cited:
**CN-A- 102 070 607** **US-A- 4 050 921**
**US-A- 4 212 870** **US-A- 5 302 605**
**US-A1- 2009 298 888** **US-A1- 2010 168 178**
**US-A1- 2010 168 178** **US-A1- 2012 122 681**
**US-A1- 2012 122 681**

- **YUANMING ZHU ET AL: "Discovery and Characterization of Sulfoxaflor, a Novel Insecticide Targeting Sap-Feeding Pests", JOURNAL OF AGRICULTURAL AND FOOD CHEMISTRY, vol. 59, no. 7, 13 April 2011 (2011-04-13) , pages 2950-2957, XP55254047, US ISSN: 0021-8561, DOI: 10.1021/jf102765x**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 866 563 B1

## Description

FIELD OF THE INVENTION

The present application concerns novel N-substituted sulfilimine and sulfoximine pyridine N-oxides and their use in controlling insects and other invertebrates. The present application also concerns pesticide compositions including these compounds, and methods of controlling insects using these compounds.

BACKGROUND OF THE INVENTION

Controlling pest populations is essential to modem agriculture, food storage, and hygiene. There are more than ten thousand species of pests that cause losses in agriculture. The worldwide agricultural losses amount to billions of U.S. dollars each year. Pests, such as termites, are also known to cause damage to all kinds of private and public structures resulting in billions of U.S. dollars in losses each year. Pests also eat and adulterate stored food, resulting in billions of U.S. dollars in losses each year, as well as deprivation of food needed for people. Certain pests have or are developing resistance to pesticides in current use. Hundreds of pest species are resistant to one or more pesticides. Accordingly, there exists a continuous need for new pesticides and for processes of forming such pesticides.

U.S. Patent Nos. 7,678,920 and 7,687,634 describe certain pesticidal sulfoximine compounds and U.S. Patent No. 8, 188,292 describes certain pesticidal sulfilimine compounds. CN 102070607 and US 5,302,605 describe pyridine N-oxides with pesticidal activity.

Some of these sulfoximine and sulfilimine compounds contain a pyridine functional group. It has now been surprisingly discovered that forms of one or more of these compounds where the pyridine functional group has been N-oxidized exhibit insecticidal properties.

SUMMARY OF THE INVENTION

One embodiment concerns compounds useful for the control of insects according to formula (I)

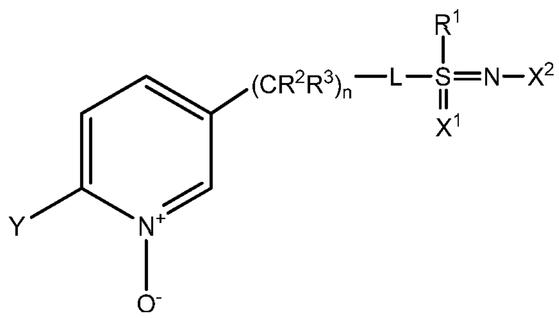

(I)

wherein

L represents a single bond or $R^1$, S and L taken together represent a 4-, 5- or 6-membered ring;
$R^1$ represents ($C_1$-$C_4$) alkyl;
$R^2$ and $R^3$ individually represent hydrogen, methyl, ethyl, flouro, chloro or bromo;
n is an integer from 0-3;
Y represents ($C_1$-$C_4$) haloalkyl, F, Cl, Br, or I;
$X^1$ is optional and represents O when present;
$X^2$ represents CN and
$R^4$ represents ($C_1$-$C_3$) alkyl.

More particular forms of compounds of formula (I) include the following classes:

(1) Compounds of formula (I) wherein $X^1$ is present and $X^2$ represents CN.
(2) Compounds of formula (I) wherein Y represents $CF_3$ or Cl.
(3) Compounds of formula (I) wherein $R^2$ and $R^3$ independently represent hydrogen, methyl or ethyl.
(4) Compounds of formula (I) wherein $R^1$ represents $CH_3$ and L represents a single bond, i.e., having the structure

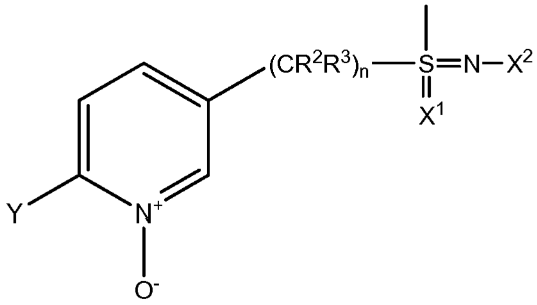

wherein n=1-3.

(5) Compounds of formula (I) wherein wherein $R^1$, S and L taken together form a saturated 5-membered ring, and n is 0, i.e., having the structure

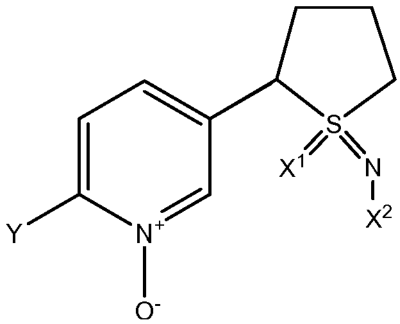

It will be appreciated by those skilled in the art that one or more combinations of the above described classes of the compound of formula (I) are possible.

Another embodiment relates to the compounds of formula (5), (6), (9), (10), (18), (19), (21) as defined in claims and examples and their use for controlling insects and invertebrates.

Further aspects, embodiments, forms, features, benefits, objects, and advantages shall become apparent from the detailed description provided herewith.

## DETAILED DESCRIPTION OF THE INVENTION

For purposes of promoting an understanding of the principles of the invention, reference will now be made to the following embodiments and specific language will be used to describe the same.

Unless specifically limited otherwise, the below listed terms as used herein shall mean the following:

"alkenyl", as used herein, means an acyclic, unsaturated (at least one carbon-carbon double bond), branched or unbranched, substituent consisting of carbon and hydrogen, for example, vinyl, allyl, butenyl, pentenyl, hexenyl, heptenyl, octenyl, nonenyl, and decenyl;

"alkoxy", as used herein, means an alkyl further consisting of a carbon-oxygen single bond, for example, methoxy, ethoxy, propoxy, isopropoxy, 1-butoxy, 2-butoxy, isobutoxy, tert-butoxy, pentoxy, 2-methylbutoxy, 1,1-dimethylpropoxy, hexoxy, heptoxy, octoxy, nonoxy, and decoxy;

"alkyl", as used herein, means an acyclic, saturated, branched or unbranched, substituent consisting of carbon and hydrogen, for example, methyl, ethyl, propyl, isopropyl, 1-butyl, 2-butyl, isobutyl, tert-butyl, pentyl, 2-methylbutyl, 1,1-dimethylpropyl, hexyl, heptyl, octyl, nonyl, and decyl;

"aryl", as used herein, means a cyclic, aromatic substituent consisting of hydrogen and carbon, for example, phenyl, naphthyl, and biphenylyl;

"halo", as used herein, means fluoro, chloro, bromo, and iodo;

"haloalkyl", as used herein, means an alkyl further consisting of, from one to the maximum possible number of, identical or different, halos, for example, fluoromethyl, difluoromethyl, trifluoromethyl, 1-fluoromethyl, 2-fluoroethyl, 2,2,2-trifluoroethyl, chloromethyl, trichloromethyl, and 1,1,2,2-tetrafluoroethyl; and

"heteroaryl", as used herein, refers to a 5- or 6-membered aromatic ring containing one or more heteroatoms, viz., N, O or S; these heteroaromatic rings may be fused to other aromatic systems.

The compounds disclosed herein can exist as one or more stereoisomers. The various stereoisomers include geometric isomers, diastereomers and enantiomers. Thus, the compounds disclosed in this document may include racemic mixtures, individual stereoisomers and optically active mixtures. It will be appreciated by those skilled in the art that one stereoisomer may be more active than the others. Individual stereoisomers and optically active mixtures may be obtained by selective synthetic procedures, by conventional synthetic procedures using resolved starting materials or by conventional resolution procedures.

One embodiment relates to insecticidal N-substituted sulfilimine and sulfoximine pyridine N-oxides according to formula (I) and their use in controlling insects and other invertebrates.

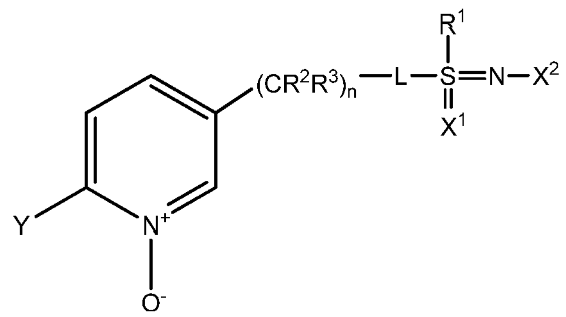

(I)

wherein

L represents a single bond or $R^1$, S and L taken together represent a 4-, 5- or 6-membered ring;
$R^1$ represents $(C_1\text{-}C_4)$ alkyl;
$R^2$ and $R^3$ individually represent hydrogen, methyl, ethyl, flouro, chloro or bromo;
n is an integer from 0-3;
Y represents $(C_1\text{-}C_4)$ haloalkyl, F, Cl, Br, or I;
$X^1$ is optional and represents O when present;
$X^2$ represents CN and
$R^4$ represents $(C_1\text{-}C_3)$ alkyl.

More particular but non-limiting forms of compounds of formula (I) include the following classes:

(1) Compounds of formula (I) wherein $X^1$ is present and $X^2$ represents CN.
(2) Compounds of formula (I) wherein Y represents $CF_3$ or Cl.
(3) Compounds of formula (I) wherein $R^2$ and $R^3$ independently represent hydrogen, methyl or ethyl.
(4) Compounds of formula (I) wherein $R^1$ represents $CH_3$ and L represents a single bond, i.e., having the structure

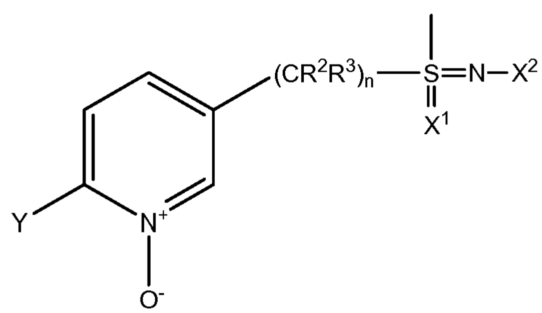

wherein n=1-3.
(5) Compounds of formula (I) wherein wherein $R^1$, S and L taken together form a saturated 5-membered ring, and n is 0, i.e., having the structure

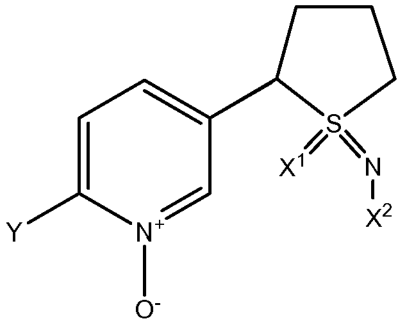

It will be appreciated by those skilled in the art that one or more combinations of the above described classes of the compound of formula (I) are possible.

Another embodiment relates to the compounds of formula (5), (6), (9), (10), (18), (19), (21) as defined in claims and examples and their use for controlling insects and invertebrates.

In one form, compounds according to formula (I) wherein Y represents ($C_1$-$C_4$) haloalkyl may be prepared in accordance with the reaction illustrated in Scheme A:

Scheme A

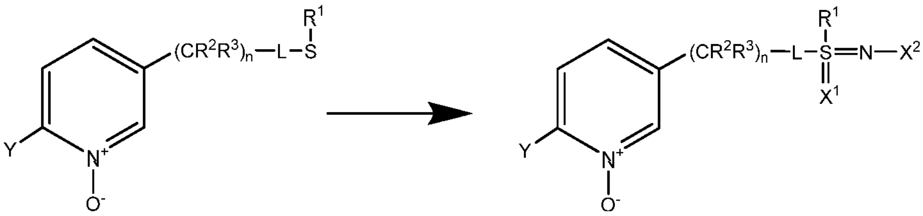

(II) (I)

Further details regarding the preparation of 2-substituted-5-(1-alkylthio)alkyl-pyridine N-oxides according to formula (II) used in Scheme A will be provided below. Depending on the desired final form of the compound according to formula (I), Scheme A may include one or more steps and is representative of the addition of $NX^2$ to a compound according to formula (II) to provide an N-substituted sulfilimine pyridine N-oxide compound according to formula (I), or the addition of both of $X^1$ and $NX^2$ to a compound according to formula (II) to provide an N-substituted sulfoximine pyridine N-oxide compound according to formula (I).

In one form, preparation of an N-substituted sulfiliminc pyridine N-oxide compound where $X^2$ represents $NO_2$ involves the reaction of a compound according to formula (II) with nitramide in the presence of acetic anhydride in Scheme A. In another form, preparation of an N-substituted sulfilimine pyridine N-oxide compound where $X^2$ represents CN involves the oxidation of a compound according to formula (II) with iodobenzene diacetate in the presence of cyanamide in Scheme A. This oxidation can be carried out in a polar aprotic solvent such as $CH_2Cl_2$. Further details regarding preparations of N-substituted sulfilimine pyridines of this nature and in which the 2-substituted-5-(1-alkylthio)alkyl-pyridine N-oxides of formula (II) of Scheme A could be used to provide corresponding N-substituted sulfilimine pyridine N-oxides are disclosed in U.S. Patent No. 8,188,292.

Preparation of N-substituted sulfoximine pyridine N-oxide compounds according to formula (I), i.e., where $X^1$ is present and represents O, may be accomplished by further oxidation of the N-substituted sulfilimine pyridine N-oxide compounds described above. For example, in one non-limiting form an N-substituted sulfilimine pyridine N-oxide compound where $X^2$ represents CN, and CN has been added by oxidation of a compound according to formula (II) with iodobenzene diacetate in the presence of cyanamide for example, may be further oxidized with meta-chloroperoxybenzoic acid (mCPBA) in the presence of a base such as potassium carbonate to provide a corresponding N-substituted sulfoximine pyridine N-oxide compound. This reaction may be carried out in protic polar solvents such as ethanol and water.

Preparation of N-substituted sulfoximine pyridine N-oxide compounds according to formula (I), i.e., where $X^1$ is present and represents O, may also be accomplished by the stepwise addition of $X^1$, N and $X^2$ to a compound according to formula (II). For example, a compound according to formula (II) may be oxidized with mCPBA in a polar solvent such as dichloromethane below 0 °C to provide a sulfoxide. The sulfoxide is subsequently iminated with sodium azide in the presence of concentrated sulfuric acid in an aprotic solvent such as chloroform under heating to provide a sulfoximine. For instances where $X^1$ is present and $X^2$ represents $NO_2$, or CN, this sulfoximine can be either nitrated with nitric acid in the presence of acetic anhydride under mildly elevated temperature, or cyanated with cyanogen bromide in the

presence of a base, to provide an N-substituted sulfoximine pyridine N-oxide. Base is required for efficient cyanation and the preferred base is DMAP, whereas sulfuric acid is used as catalyst for efficient nitration reaction. Further details regarding preparations of N-substituted sulfoximine pyridines of this nature and in which the 2-substituted-5-(1-alkylthio)alkyl-pyridine N-oxides of formula (II) of Scheme A could be used to provide corresponding N-substituted sulfoximine pyridine N-oxides are disclosed in U.S. Patent Nos. 7,678,920 and 7,687,634.

Certain compounds according to formula (II) which may be used in the reaction illustrated in Scheme A may be obtained by condensing an enamine with an α, β-unsaturated ketone to provide an intermediate compound, and the intermediate compound is cyclized using an amine nucleophile. More particularly, one non-limiting process for the preparation of a 2-substituted-5-(1-alkylthio)alkyl-pyridine N-oxide according to formula (II)

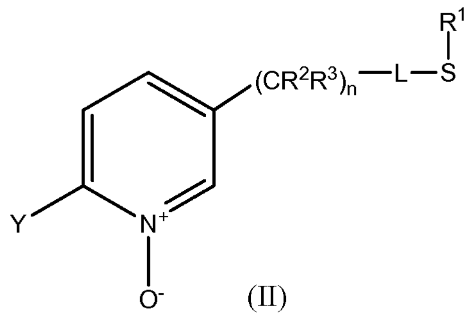

(II)

wherein Y represents ($C_1$-$C_4$) haloalkyl and L, $R^1$, $R^2$, $R^3$ and n are as previously defined utilizes the approach illustrated in Scheme B:

Scheme B

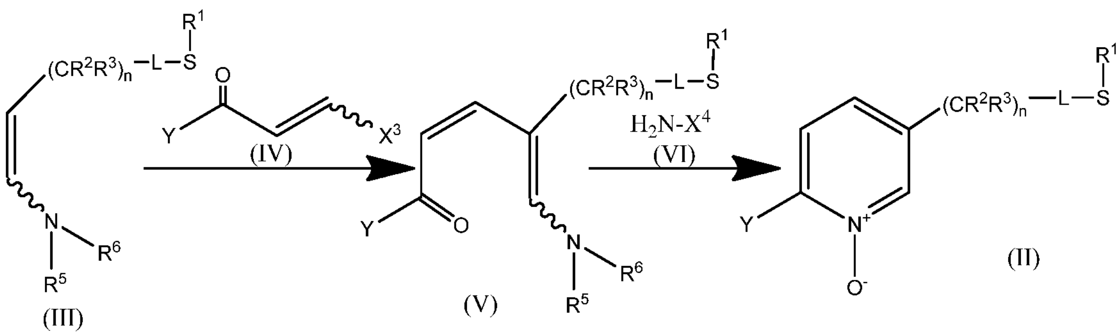


(III) (V) (II)

In Scheme B, an enamine according to formula (III)

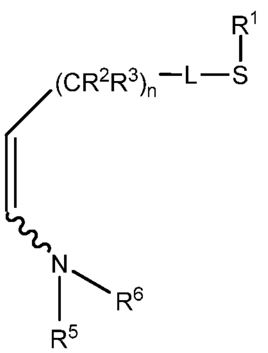

(III)

wherein

$R^1$, $R^2$, $R^3$, L, and n are as previously defined; and

$R^5$ and $R^6$ independently represent $C_1$-$C_8$ alkyl, $C_2$-$C_8$ alkenyl, $C_1$-$C_8$ arylalkyl, $C_1$-$C_8$ haloalkyl, $C_1$-$C_8$ alkoxyalkyl,

$C_1$-$C_8$ alkylaminoalkyl, aryl, or heteroaryl or $R^5$ and $R^6$ taken together with N represent a 5- or 6-membered saturated or unsaturated ring;

is condensed with an α, β-unsaturated ketone according to formula (IV)

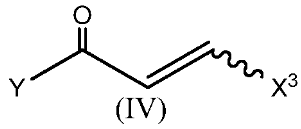

(IV)

wherein

Y represents ($C_1$-$C_4$) haloalkyl; and
$X^3$ represents halogen, $OR^7$, $OSO_2R^7$, $SR^7$, $SOR^7$, $SO_2R^7$ or $NR^8R^9$, where $R^7$ represents hydrogen, $C_1$-$C_8$ alkyl, $C_2$-$C_8$ alkenyl, $C_1$-$C_8$ arylalkyl, $C_1$-$C_8$ haloalkyl, $C_1$-$C_8$ alkoxyalkyl, $C_1$-$C_8$ alkylaminoalkyl, aryl or heteroaryl, and $R^8$ and $R^9$ independently represent hydrogen, $C_1$-$C_8$ alkyl, $C_2$-$C_8$ alkenyl, $C_1$-$C_8$ arylalkyl, $C_1$-$C_8$ haloalkyl, $C_1$-$C_8$ alkoxyalkyl, $C_1$-$C_8$ alkylaminoalkyl, aryl or heteroaryl, or $R^8$ and $R^9$ taken together with N represent a 5-or 6-membered saturated or unsaturated ring;

to provide an intermediate compound according to formula (V)

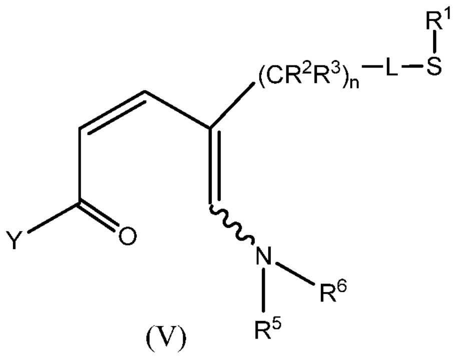

(V)

wherein Y represents ($C_1$-$C_4$) haloalkyl and $R^1$, $R^2$, $R^3$, $R^5$, $R^6$, L and n are as previously defined.

As also illustrated in scheme B, the intermediate compound according to formula (V) is cyclized using an amine nucleophile according to formula (VI)

$H_2N$-$X^4$ (VI)

wherein $X^4$ represents hydroxyl, alkoxy, cyano, amino or mercaptan, under refluxing conditions to provide a compound according to formula (II).

Enamines according to formula (III) can be conveniently prepared from the addition of a suitably substituted amine to an appropriately substituted aldehyde in the presence of a water adsorbing material, with or without a suitable solvent. Typically, the appropriately substituted aldehyde is reacted with an anhydrous di-substituted amine at about -20 °C to about 20 °C in the presence of a desiccant such as anhydrous potassium carbonate, and the product is isolated by routine procedures and usually used without further purification. In one non-limiting form for example where the enamine according to formula (III) has the following structure

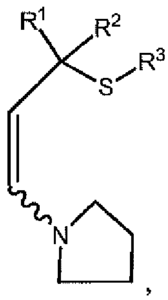

the appropriately substituted aldehyde is reacted with pyrrolidine at about -20 °C to about 20 °C in the presence of a desiccant such as anhydrous potassium carbonate, and the resulting product is isolated by routine procedures and usually used without further purification. Further details regarding the production of enamines according to formula (III) are found, for example, in U.S. Patent Publication No. 2008/0033180.

α,β-unsaturated ketones according to formula (IV) are commercially available or can be prepared from the corresponding vinylogous substrates and acylating agents. In one form for example, alkylvinyl ethers can be acylated with haloalkylacetic anhydrides to yield compounds according to formula (IV).

Approximately equimolar quantities of the enamine according to formula (III) and the α, β-unsaturated ketone according to formula (IV) are required in the condensation process.

In one form, the condensation is conducted at a temperature from about -20 °C to about 35 °C. In another more particular form, temperatures from about -5 °C to about 20 °C are used.

The condensation of the enamine according to formula (III) with the α, β-unsaturated ketone according to formula (IV) may be conducted in a polar or non-polar solvent, although forms in which it is conducted in solvent-free conditions are also contemplated. Non-limiting examples of polar solvents include dichloromethane, tetrahydrofuran, ethyl acetate, acetone, dimethylformamide, acetonitrile, and dimethyl sulfoxide, while non-limiting examples of non-polar solvents include hydrocarbon and aromatic hydrocarbon solvents such as toluene. In one particular but non-limiting form, this condensation is conducted in toluene.

In one aspect, the α, β-unsaturated ketone according to formula (IV) is added to a preformed mixture of the enamine according to formula (III).

In a typical condensation reaction, the enamine according to formula (III) is dissolved in the desired solvent at about -5°C to about 20°C and the α, β-unsaturated ketone according to formula (IV) is continuously added via addition funnel to this solution. The mixture is agitated until the enamine according to formula (III) and the α, β-unsaturated ketone according to formula (IV) are consumed. With the use of a non-polar solvent such as toluene, the intermediate compound according to formula (V) can be used as is without further isolation or purification.

The cyclization of the intermediate compound according to formula (V) with an amine nucleophile according to formula (VI) is performed under refluxing conditions; i.e., at a temperature in the range of 50 °C to 90 °C. As indicated above, $X^4$ may represent hydroxyl, alkoxy, cyano, amino or mercaptan. It is also possible for the amine nucleophile used in reaction Scheme B to be present in the form of an acid salt. When an acid salt form of the amine nucleophile is used, a non-nucleophilic base is also used to neutralize the acid salt analog. Non-limiting examples of non-nucleophilic bases include carbonate salts, triethylamine, N,N-diisopropylethylamine, and 1,8-diazabicycloundec-7-ene. In one non-limiting form where $X^4$ represents hydroxyl and the compound according to formula (VI) is hydroxylamine, hydroxylamine hydrochloride is used in reaction Scheme B along with triethylamine. Still, it should be appreciated that other variations in the amine nucleophile according to formula (VI) and the non-nucleophilic base, when present, are possible and contemplated.

The cyclization of the intermediate compound according to formula (V) may be conducted in the same solvent as the condensation of the enamine according to formula (III) and the α, β-unsaturated ketone according to formula (IV).

Forms of the intermediate compound according to formula (V) where Y represents ($C_1$-$C_4$) haloalkyl and $R^1$, $R^2$, $R^3$, $R^5$, $R^6$, L and n are as previously defined may also be prepared utilizing the approach illustrated in Scheme C:

Scheme C

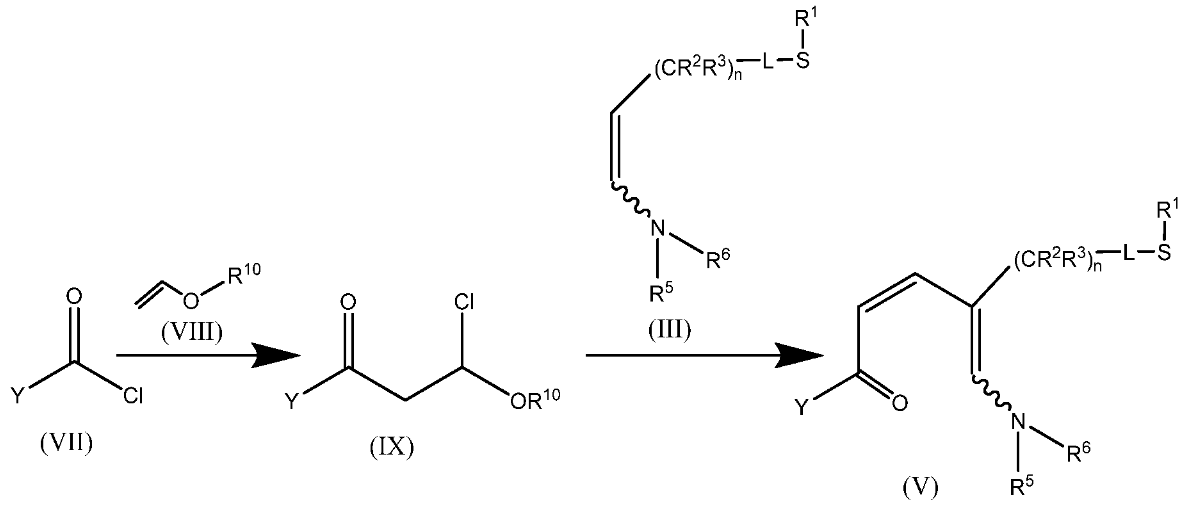

(VII) (IX) (V)

In Scheme C, an acetyl chloride compound according to formula (VII) where Y represents $C_1$-$C_4$ haloalkyl is reacted with an alkyl vinyl ether according to formula (VIII) where $R^{10}$ represents $C_1$-$C_4$ alkyl. Approximately equimolar quantities of compounds according to formulas (VII) and (VIII) are generally used in the process, although excesses of one or the other may be employed. In one particular form, a 10-50 percent stoichiometric excess of the alkyl vinyl ether according to formula (VIII) is utilized.

This reaction is conducted either in the absence of a solvent, e.g., with excess of the alkyl vinyl ether according to formula (VIII), or in the presence of an anhydrous organic solvent. Non-limiting examples of suitable solvents are hydrocarbon solvents, including aromatic hydrocarbons such as toluene. The reaction may be conducted at a temperature from about -10 °C to about 35 °C. In one particular form, temperatures from about 0 °C to about 20 °C are used. In a typical reaction, the acetyl chloride compound according to formula (VII) is bubbled below the surface of the alkyl vinyl ether compound according to formula (VIII), either neat or in the presence of a hydrocarbon solvent, between 0-5 °C. The reaction is allowed to warm with stirring for about 1 hour, keeping the temperature no higher than room temperature. The crude reaction mixture containing the intermediate compound according to formula (IX) may be used as is without further isolation or purification of the reaction mixture.

The intermediate compound according to formula (IX) is then condensed with an enamine according to formula (III) in the presence of a tertiary amine base to provide an intermediate compound according to formula (V) where Y represents $C_1$-$C_4$ haloalkyl. Approximately equimolar quantities of the intermediate compound according to formula (IX) and the enamine according to formula (III) are required in the condensation process; at least one equivalent of tertiary amine base is required with between about 1 and about 2 equivalents being utilized in certain forms.

This condensation may be conducted at a temperature from about -20 °C to about 35 °C. In one particular form, temperatures from about -5 °C to about 20 °C are utilized. This condensation may be conducted in a non-polar or polar aprotic solvent. Exemplary non-polar solvents include hydrocarbon solvents and aromatic hydrocarbons. Polar aprotic solvents are also a good choice for this chemistry. Either acetonitrile or toluene is used in particular but non-limiting forms. In one form, the intermediate compound according to formula (IX) is added to a preformed mixture of the enamine according to formula (III) and a tertiary amine base. In a typical condensation reaction, the enamine according to formula (III) and at least a stoichiometric amount of a tertiary amine base are dissolved in the desired solvent at about - 50 °C to about 200 °C and the intermediate compound according to formula (IX) is continuously added via addition funnel to this solution. The mixture is agitated until the intermediate compound according to formula (IX) and the enamine according to formula (III) are consumed. The intermediate compound according to formula (V) may be used as is without further isolation or purification. Further details regarding the approach illustrated in Scheme C are provided in International Patent Publication No. WO 2010/002577.

The intermediate compound according to formula (V) prepared by this approach may be cyclized using an amine nucleophile according to formula (VI) as discussed above.

More particular but non-limiting forms of compounds of formula (II) include the following classes:

(1) Compounds of formula (II) wherein Y is $CF_3$.
(2) Compounds of formula (II) wherein $R^2$ and $R^3$ independently represent hydrogen, methyl or ethyl.
(3) Compounds of formula (II) wherein $R^1$ represents $CH_3$ and L represents a single bond, i.e., having the structure

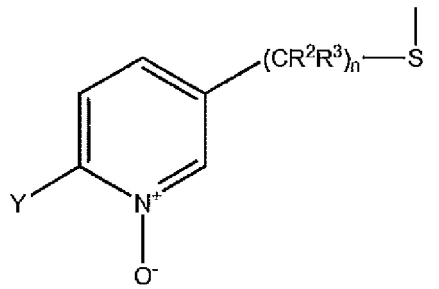

wherein n=1-3.

(4) Compounds of formula (II) wherein $R^1$, S and L taken together form a saturated 5-membered ring, and n is 0, i.e., having the structure

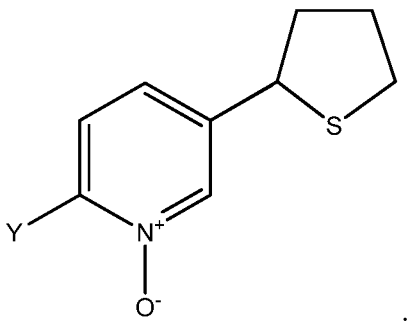

.

It will be appreciated by those skilled in the art that one or more combinations of the above described classes of the compound of formula (I) are possible.

In another form, sulfoximine pyridine N-oxide compounds according to formula (I), i.e., where $X^1$ is present and represents O, may be prepared by oxidizing compounds according to formula (X)

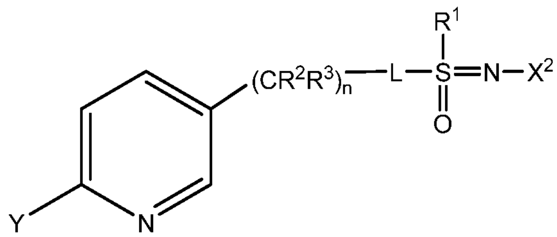

(X),

wherein Y represents ($C_1$-$C_4$) haloalkyl, F, Cl, Br, or I and $X^2$, L, $R^1$, $R^2$, $R^3$, n, and $R^4$ are as previously defined, by the addition of urea hydrogen peroxide and trifluoroacetic anhydride. This reaction is illustrated in Scheme D:

Scheme D

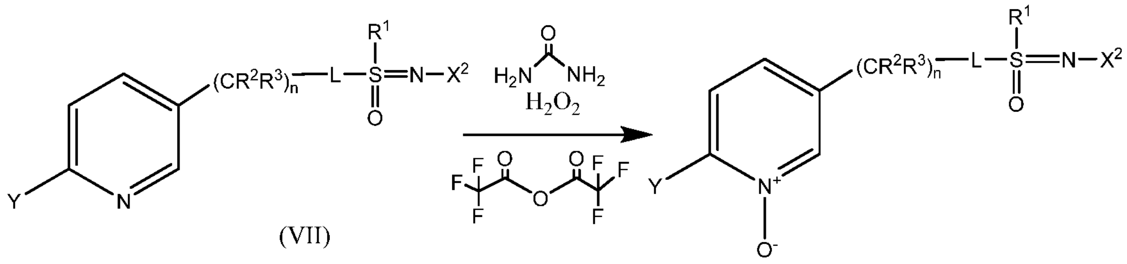

(VII)

In one form, the oxidation is conducted at a temperature from about 0 °C to about 30 °C. In another exemplary form, the oxidation can be carried out at room temperature and ambient pressure, but the use of higher or lower temperatures and pressures, if desired, is contemplated.

Non-limiting examples of solvents which can be used include polar solvents such as dichloromethane, tetrahydrofuran, ethyl acetate, acetone, dimethylformamide, acetonitrile, and dimethyl sulfoxide.

In one form, the compound of formula (X) is mixed with urea hydrogen peroxide and the solvent and stirred. Trifluoroacetic anhydride is then added to the resultant mixture followed by additional stirring until all or a major portion of the starting materials have been consumed. The reaction mixture may then be filtered, washed and concentrated in vacuo. The remaining residue is then taken up in a suitable solvent, such as THF (tetrahydrofuran), and washed, and the organic phase is dried, filtered and concentrated in vacuo to provide the respective sulfoximine pyridine N-oxide compound according to formula (I). It should be appreciated however that the foregoing steps are not limiting, and that variations and additions to the same are possible and contemplated.

Further details regarding the preparation of compounds of formula (X) wherein $X^2$ is $NO_2$, or CN, Y represents ($C_1$-$C_4$) haloalkyl, F, Cl, Br, or I, and L, $R^1$, $R^2$, $R^3$, n and $R^4$ are as previously defined are found in U.S. Patent Nos. 7,678,920 and 7,687,634.

EXAMPLES

The examples are for illustration purposes and are not to be construed as limiting the invention disclosed in this document to only the embodiments disclosed in these examples.

Starting materials, reagents and solvents which were obtained from commercial sources were used without further purification. Molecules are given their known names, named according to naming programs within ISIS Draw, ChemDraw or ACD Name Pro. If such programs are unable to name a molecule, the molecule is named using conventional naming rules. Unless otherwise specified, $^1$H and $^{13}$C NMR spectra were performed using a Bruker 300 MHz instrument; gas Chromatography was performed using an Agilent 6850 Network GC system or on a Agilent 6890 with the ability for cold on column injections with a capillary column; and HPLC was performed using an Agilent 1200 system containing an autosampler, vacuum degasser, column heater, and UV detection.

**Example 1: Small Scale Preparation of 5-(1-(methylthio)ethyl)-2-(trifluoromethyl)pyridine N-oxide (1):**

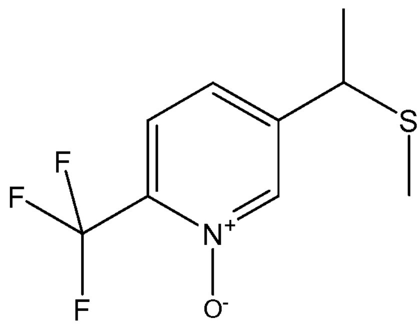

(1)

A condensation reaction of 1-(3-methylthiobut-1-enyl)pyrrolidone (2)

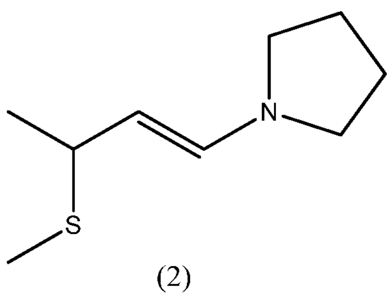

(2)

with 4-ethoxy-1,1,1-trifluorobut-3-en-2-one (3)

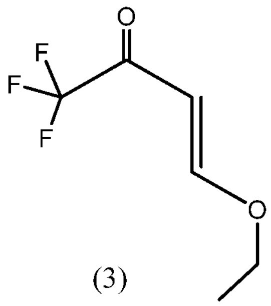

(3)

in toluene yielded a 27 wt% 1,1,1-trifluoro-6-(methylthio)-5-(pyrrolidine-1-ylmethylene)hept-3-en-2-one (4) in toluene

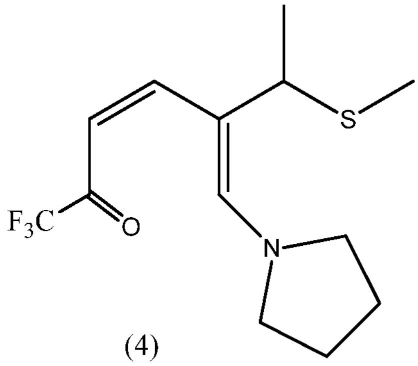

(4)

.

403 mg (0.37 mmol) of the 27 wt% 1,1,1-trifluoro-6-(methylthio)-5-(pyrrolidine-1-ylmethylene)hept-3-en-2-one (4) in toluene was added to a 25 mL three-neck round bottom flask equipped with a reflux condenser and vented to a bleach scrubber. To this mixture was added 34 mg (0.34 mmol) of triethylamine in one portion. The reaction mixture was cooled to about 12.8 °C and then 24 mg (0.34 mmol) of hydroxylamine hydrochloride was added in one portion. The reaction mixture was slowly heated to 85 °C and stirred for one hour and forty-five minutes. The reaction mixture was then cooled to ambient temperature. This mixture was split into many fractions for instrumental analysis and purification. A portion of the reaction mixture was partitioned between toluene and water. Both the organic and aqueous layers were analyzed by LC/MS. Both layers were confirmed to have a peak with molecular consistency for 5-(1-(methylthio)ethyl)-2-(trifluoromethyl)pyridine N-oxide ($C_9H_{10}F_3NOS$). Calculated m/z = 237.04. Found m/z = 237.04.

A small portion of the reaction mixture was purified using preparatory thin layer chromatography by loading 2 mL of the reaction mixture onto a 20 cm by 20 cm plate (1000 microns) and eluting it with a mixture having a ratio of 4:1 between hexanes and 2-propanol (Rf was about 0.5 to 0.6). The appropriate band was cut from the plate and extracted off of the silica gel with 20 mL of ethyl acetate. A proton NMR was taken of the best fractions of this separation. The material contained a small portion of ethyl acetate, but the chemical shifts for the desired compound are: $^1H$ NMR (300 MHz, $CDCl_3$) $\delta$ 8.30 (s, 1H), 7.64 (d, *J* = 8.3 Hz, 1H), 7.35 (d, *J* = 8.3 Hz, 1H), 3.78 (q, *J* = 7.1 Hz, 1H), 1.98 (s, 3H), 1.58 (d, *J* = 7.1 Hz, 3H).

**Example 2: Larger scale preparation of 5-(1-(methylthio)ethyl)-2-(trifluoromethyl)pyridine N-oxide (1):**

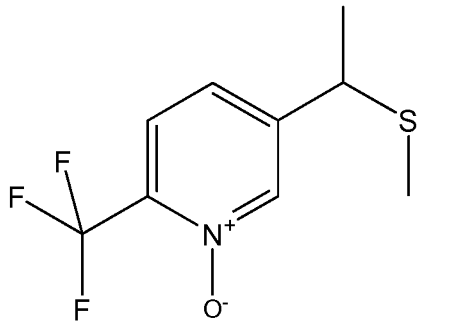

(1)

5.0 g (0.03 moles) of 1-(3-methylthiobut-1-enyl)pyrrolidone (2) and 100 mL of acetonitrile (ACN) were added to a dry 250 mL round-bottom flask equipped with a magnetic stirrer, nitrogen inlet, addition funnel, and reflux condenser. 4-ethoxy-1,1,1-trifluorobut-3-en-2-one (3) (ETFBO) (4.9 g, 0.03 moles) was then added dropwise over 2-3 minutes, and a resulting dark solution was stirred at room temperature for 1 hour. 2.1 g (0.03 moles) of hydroxyl amine hydrochloride was then added to this solution followed by 4.2 mL (0.03 moles) of triethylamine. The reaction was then refluxed at 85 °C for 2 hours, cooled, and an aliquot was analyzed by TLC and GC/MS which showed that the reaction was essentially complete, no starting material remained, and the existence of two new products. The major product identified upon analysis by GC/MS was consistent with the structure assigned to 5-(1-(methylthio)ethyl)-2-(trifluoromethyl)pyridine N-oxide (1), and the minor product appeared to be the trans-amination product of ETFBO and pyrrolidine. The reaction mixture was then stirred at room temperature for 12 hours, poured into about 100 mL of water and extracted with three 100 mL volumes of ethyl ether. The ether extract was washed with water and saturated aqueous sodium chloride solution, dried over anhydrous magnesium sulfate, filtered and concentrated under vacuum on a rotary evaporator. The crude product (6.1 g) was chromatographed on silica gel with a gradient of 100% hexane to 100% ethyl acetate over 20 minutes. Isolated 2.2 g of a yellow liquid which was consistent with the structure assigned to 5-(1-(methylthio)ethyl)-2-(trifluoromethyl)pyridine N-oxide (1) upon analysis by 300 MHz $^1H$ NMR and GC/MS; 31% isolated yield. $^1H$ NMR (300 MHz, Chloroform-d) $\delta$ 8.28 (s, 1H), 7.63 (d, J = 8.3 Hz, 1H), 7.34 (d, *J* = 8.3 Hz, 1H), 3.77 (q, J = 7.1 Hz, 1H), 1.98 (s, 3H), 1.56 (d, J = 7.3, 3H). Calculated m/z = 237.04. Found m/z = 237.04.

**Example 3: Preparation of N-Cyano-S-[1-(6-trifluoromethyl-3-pyridinyl)ethyl]-S-methylsulfilimine N-oxide (5):**

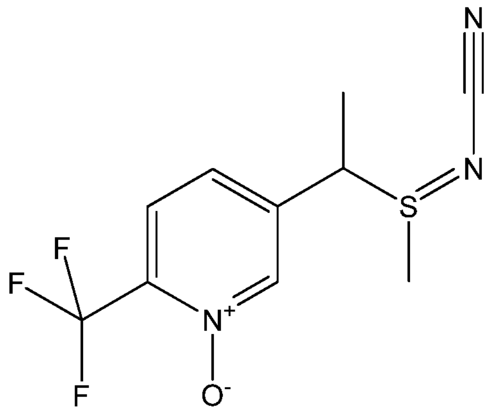

(5)

2.2 g (0.0092 moles) of 5-(1-(methylthio)ethyl)-2-(trifluoromethyl)pyridine N-oxide (1), 0.38 g (0.0092 moles) of cyanamide and 100 mL of anhydrous tetrahydrofuran (THF) were added to a dry 250 mL round-bottom flask equipped with a magnetic stirrer, nitrogen inlet, and thermometer. The solution was cooled to about 4 °C, and iodobenzene diacetate (3.0 g, 0.0092 moles) was added in one portion. The reaction was stirred at 0-4 °C for 2 hours, allowed to warm gradually to room temp, and then stirred at ambient temperature under nitrogen. After 13 hours, an aliquot of the reaction mixture was analyzed by HPLC using a YMC AQ column (Kyoto, Japan) with a 1.0 mL/min flow rate. Acetontitrile (ACN) and

water with 0.05% trifluoroacetic acid (TFA) were used as solvents. A linear gradient was used starting at 20% ACN/80% water with 0.05% TFA and transitioning to 95% ACN/5% water with 0.05% TFA over 25 minutes. The HPLC analysis indicated that the reaction was essentially complete. The reaction mixture was then diluted with about 200 mL of ACN and washed with two 100 mL volumes of hexanes to remove the iodobenzene byproduct. The ACN solution was concentrated under vacuum on a rotary evaporator, and the resulting crude product was chromatographed on silica gel with a gradient of 50% hexanes/50% acetone that was transitioned to 100% acetone over 20 minutes. The pure fractions were combined, and concentrated under vacuum on a rotary evaporator to afford 1.7 g of a yellow solid which was consistent with the structure assigned to N-Cyano-S-[1-(6-trifluoromethyl-3-pyridinyl)ethyl]-S-methylsulfilimine N-oxide (5) upon analysis by 300 MHz $^1$H NMR and HPLC/MS (mix of isomers). Found: $^1$H NMR (300 MHz, DMSO-d6) δ 8.61 (dd, *J* = 34.8, 1.4 Hz, 1H), 8.03 (dd, *J* = 8.4, 4.2 Hz, 1H), 7.81 - 7.44 (m, 1H), 4.62 (p, *J* = 7.0 Hz, 1H), 2.75 (d, *J* = 19.9 Hz, 3H), 1.71 (dd, *J* = 7.2, 2.6 Hz, 3H). ESI MS (*m*/*z*) 278 [M+H]$^+$. MP = 139-141 °C (d).

**Example 4: Preparation of N-Cyano-S-[1-(6-trifluoromethyl-3-pyridinyl)ethyl]-S-methylsulfoximine N-oxide (6):**

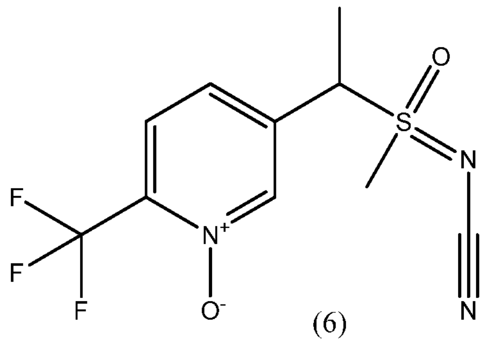

(6)

1.3 g (4.7 mmoles) of N-Cyano-S-[1-(6-trifluoromethyl-3-pyridinyl)ethyl]-S-methylsulfilimine N-oxide (5) and 100 mL of methylene chloride were added to a dry 250 mL round-bottom flask equipped with a magnetic stirrer, nitrogen inlet, addition funnel, thermometer, and reflux condenser. The solution was cooled to 10 °C and 1.7 mL of a 40 wt% sodium permanganate in water solution was added dropwise at a rate that maintained the temperature below 40 °C. After this addition was complete, the reaction was stirred at 5 °C for 30 minutes, and allowed to warm to room temperature. HPLC analysis of an aliquot of the reaction mixture indicated that the reaction was essentially complete. The solution was then filtered through filter paper, and the filtrate was washed with sodium bisulfite solution and water. The MDC solution was then dried with anhydrous magnesium sulfate, filtered and concentrated under vacuum on a rotary evaporator. 120 mg of a yellow oil was isolated, and HPLC/MS analysis indicated that it contained a little of the desired product. Based on this analysis, the desired product appears to have poor solubility in MDC. The filter paper from the initial filtration was extracted in about 200 mL of acetone. This extract was then dried over anhydrous $MgSO_4$, filtered and stripped. A sticky yellow solid was isolated and chromatographed on silica gel with a gradient of 25% hexanes/75% acetone transitioning to 100% acetone over 20 minutes The pure fractions were combined, and stripped to afford 74.1 mg of a white solid which was consistent with the structure assigned to N-Cyano-S-[1-(6-trifluoromethyl-3-pyridinyl)ethyl] -S-methylsulfoximine N-oxide (6) upon analysis by 300 MHz $^1$H NMR and HPLC/MS. Found: $^1$H NMR (300 MHz, DMSO-d6) δ 8.39 (d, *J* = 1.7 Hz, 1H), 7.85 (d, *J* = 8.5 Hz, 1H), 7.55 (d, *J* = 8.5 Hz, 2H), 4.83 (qd, *J* = 7.1, 2.6 Hz, 1H), 3.25 (d, *J* = 8.0 Hz, 3H), 1.98 - 1.76 (m, 3H). ESI MS (*m*/*z*) 294 [M+H]$^+$. MP = 228-231 °C.

**Examples 5-6:**

Compounds (9) and (10) of Examples 5 and 6, respectively, are shown in Table 1 below. Compounds (7) and (8) (also shown in Table 1 below) were prepared pursuant to reaction Scheme B illustrated above and utilizing processes similar to those described above in connection with Examples 1 and 2. Compounds (9) and (10) were then prepared from compounds (7) and (8), respectively, utilizing processes similar to those described above in connection with Examples 3 and 4.

TABLE 1

| Starting Compounds | Final Compounds |
|---|---|
| 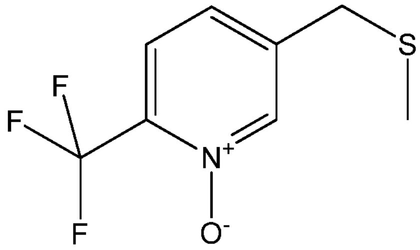 (7) | 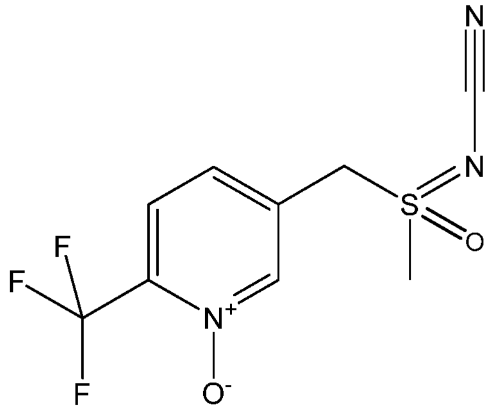 (9)<br>Found: $^1H$ NMR (300 MHz, DMSO-d6) δ 8.53 (s, 1H), 8.07 (d, *J* = 8.4 Hz, 1H), 7.58 (d, *J* = 8.5 Hz, 1H), 5.18 (s, 2H), 3.51 (s, 3H). ESI MS (m/z) 282 [M+H]+. |
| 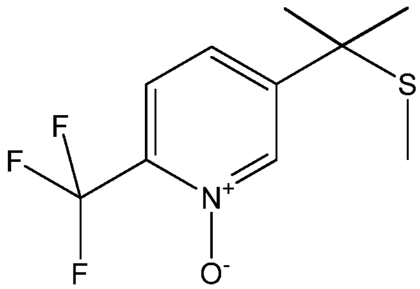 (8) | 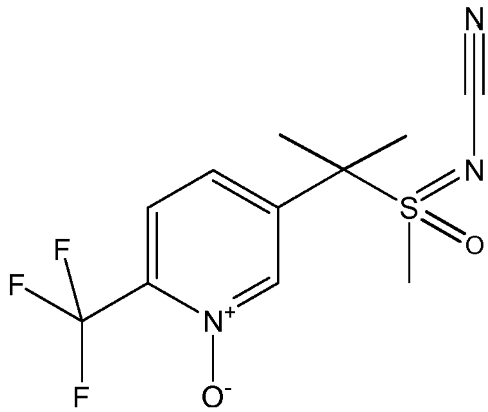 (10)<br>Found: $^1H$ NMR (400 MHz, DMSO-d6) δ 8.65 (s, 1H), 8.02 (d, *J* = 8.6 Hz, 1H), 7.73 (s, 1H), 3.41 (s, 3H), 3.41 (s, 3H), 1.92 (s, 6H). ESI MS (m/z) 308 [M-H]-. |

**Example 7:**

Compound (11) of Example 7 is shown in Table 2 below. Compound (6) was acid hydrolyzed utilizing a process similar to that described herein above and in connection with Example 10 below to provide compound (11).

TABLE 2

| Starting Compound | Acid hydrolysed (Reference) |
|---|---|

(continued)

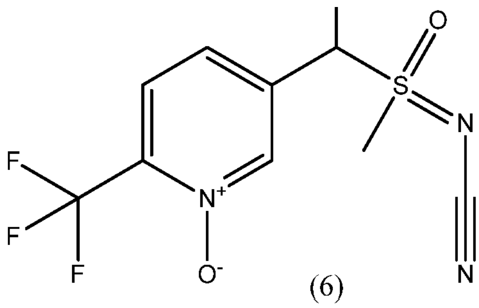

(6)

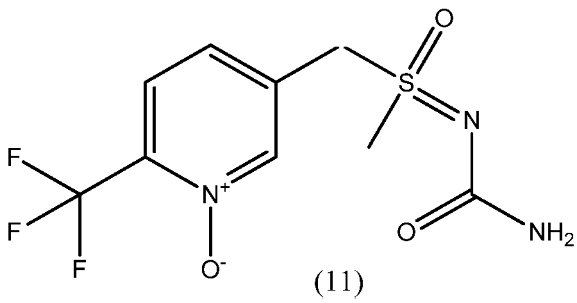

(11)

Found: $^1$H NMR (300 MHz, DMSO-d6) δ 11-10 (bs, 2H) 8.53 (dd, $J$ = 3.9, 1.4 Hz, 1H), 7.97 (dd, $J$ = 8.4, 5.4 Hz, 1H), 7.73 - 7.48 (m, 1H), 4.99 (dq, $J$ = 14.2, 7.1 Hz, 1H), 3.18 (d, $J$ = 4.6 Hz, 3H), 1.82 - 1.49 (m, 3H). ESI MS (m/z) 312 [M+H]+.

**Example 8: Preparation of [methyl(oxido){1-[1-oxido-6-(trifluoromethyl)pyridin-3-yl]ethy}-λ$^6$-sulfanylidene]cyanamide (6):**

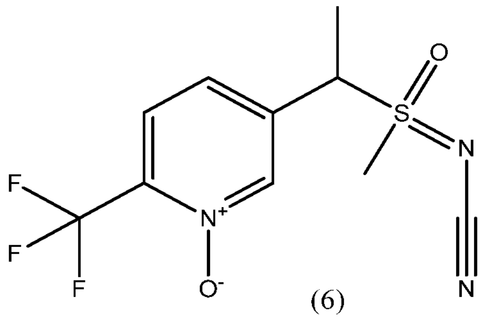

(6)

Trifluoroacetic anhydride (TFAA) was added dropwise to a mixture of {1-[6-(trifluoromethyl)pyridin-3-yl]ethyl}(methyl)oxido-λ$^4$-sulfanylidenecyanamide (12) and urea hydrogen peroxide in 10 mL of acetonitrile, under $N_2$, at room temperature. {1-[6-(trifluoromethyl)pyridin-3-yl]ethyl}(methyl)oxido-λ$^4$-sulfanylidenecyanamide (12) is commonly known as sulfoxaflor and has the following structure

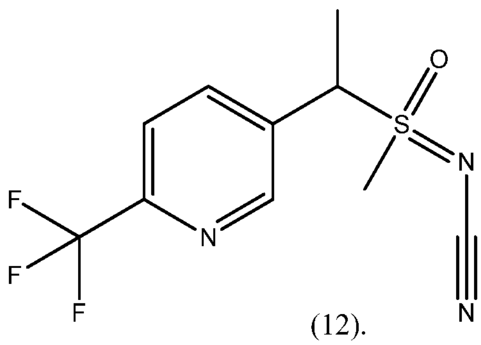

(12).

An exothermic output of about 8 °C was observed and the mixture became homogeneous as TFAA was added. After stirring at room temperature for 60 minutes, thin layer chromatography (TLC) analysis conducted with a 1:1 ratio of hexanes to acetone showed about a 2:1 ratio of compound (6) to compound (12). After 2 hours, TLC analysis under conditions indicated above showed only a minor amount of compound (12) remaining. After stirring at room temperature for 3 hours, a white precipitate was present and the reaction mixture was filtered through a medium fritted funnel, washing with $CH_3CN$ (acetonitrile). The resulting filtrate was then concentrated in vacuo. The residue was taken up in 30 mL of

THF, and washed with two 10 mL volumes of saturated sodium thiosulfate and one 10 mL volume of saturated NaCl. The resulting organic phase was dried with $Na_2SO_4$/$MgSO_4$, filtered and concentrated in vacuo to give 0.82 g of a light yellow wax. The crude material was dissolved in acetone and chromatographed by flash chromatography using a Teledyne-Isco CombiFlash Companion® (Isco, Inc., Lincoln, Nebraska) flash chromatography system equipped with a 40 g RediSep silica gel column (Isco, Inc.). The chromatography was performed with a flow rate of 40 mL/minute, detection at 254 nm (monitored at 280 nm), and hexanes and acetone used as solvents. A linear gradient was used starting at 75% hexanes/25% acetone for two minutes and transitioning to 100% acetone over a period of 14 minutes and then held at 100% acetone for 8 minutes. Isolation of the major product gave 0.178 g (34% yield) of compound (6) as a light tan solid. $^1$H NMR (DMSO-$d_6$) d 8.58 (s, 1H), 8.03 (d, 1H, $J$ = 8.4), 7.61 (d, 1H, $J$ = 8.4), 5.22 (q, 1H, $J$ = 7.2), 3.38/3.46 (two singlets, 3H), 1.80 (d, 3H, $J$ = 7.2). MS: (ES$^+$) 294 (M+H); (ES$^-$) 292 (M-H).

**Example 9: Preparation of [methyl(oxido){[1-oxido-6-(trifluoromethyl)pyridin-3-yl]methyl}-λ$^6$-sulfanylidene]cyanamide (9):**

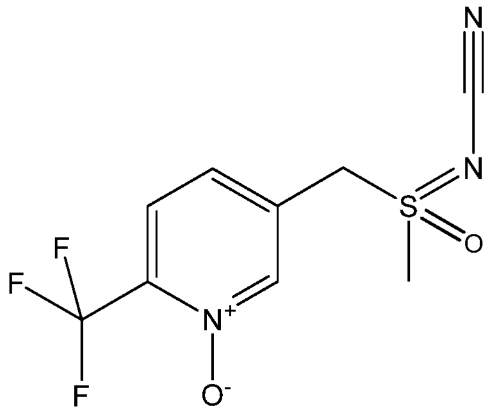

(9)

A 50 mL, three neck round bottom flask equipped with a magnetic stir bar and thermometer was charged with [(6-trifluoromethylpyridin-3-yl)methyl](methyl)oxido-λ$^4$-sulfanylidenecyanamide (13), $CH_2Cl_2$ and urea hydrogen peroxide. [(6-trifluoromethylpyridin-3-yl)methyl](methyl)oxido-λ$^4$-sulfanylidenecyanamide (13) has the following structure:

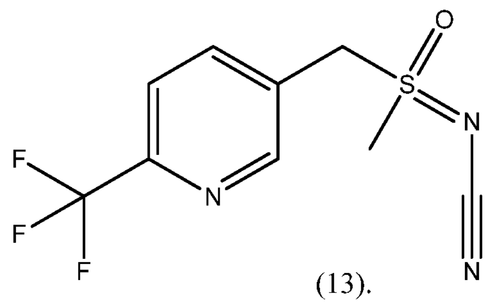

(13).

The resultant mixture was cooled in an ice bath to less than 5 °C and treated dropwise with trifluoroacetic anhydride (TFAA). An exothermic output of about 8 °C was observed as TFAA was added. After the mixture was kept for 60 minutes at 0-5 °C, thin layer chromatography (TLC) analysis conducted with a 1:1 ratio of hexanes to acetone showed that compound (13) was mainly present. The reaction mixture was gradually allowed to warm to room temperature. After stirring at room temperature, TLC analysis was performed again and showed that compound (13) was mainly present. The reaction mixture was treated with 3 mL of anhydrous $CH_3CN$ (acetonitrile) in an effort to dissolve insoluble material that was present in the reaction mixture. After about 3 hours, TLC analysis was performed again and showed a mixture of about a 3:1 ratio of compound (13) to what was assumed to be compound (9) given the presence of several minor products. After stirring at room temperature for 3 days, further TLC analysis indicated that none of compound (13) remained in the reaction mixture. In addition, there appeared to be a minor amount of compound (9) and a greater

amount of a very polar material believed to be *N*-oxide-urea. The reaction mixture was filtered through a medium fritted funnel, washing with $CH_2Cl_2$ (dichloromethane). The resulting filtrate was concentrated in vacuo, diluted with 30 mL of THF and washed with two 10 mL volumes of saturated sodium thiosulfate. The resulting organic phase was dried with $Na_2SO_4$, filtered and concentrated in vacuo to give 1.33 g of a yellow oil. The crude material was dissolved in acetone and chromatographed by flash chromatography using a Teledyne-Isco CombiFlash Companion® (Isco, Inc., Lincoln, Nebraska) flash chromatography system equipped with a 40 g RediSep silica gel column (Isco, Inc.). The chromatography was performed with a flow rate of 40 mL/minute, detection at 280 nm (monitored at 254 nm), and hexanes and acetone used as solvents. A linear gradient was used starting at 75% hexanes/25% acetone for two minutes and transitioning to 100% acetone over a period of 14 minutes and then held at 100% acetone for 8 minutes. Isolation of compound (9) gave 75 mg (14% yield) of a light tan solid with the following 8.1), 7.52 (d, 1H, *J* = 8.1), 4.63 (s, 2H), 3.02 (s, 3H); MS ($ES^-$) 278 (M-H).

**Example 10: Preparation of 1-[methyl(oxido){1-[1-oxido-6-(trifluoromethyl)pyridin-3-yl]ethyl}-λ⁶-sulfanylidene] urea (11):**

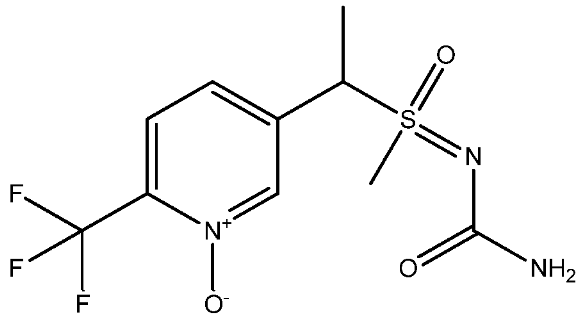

(11) Reference

A mixture of compound (6) (the production of which is described in Examples 4 and 8 above) in 5 mL of acetonitrile was treated with two drops of concentrated sulfuric acid. After stirring at room temperature for about 30 minutes, thin layer chromatography (TLC) analysis conducted with a 1:1 ratio of hexanes to acetone showed only compound (6) present in an aliquot diluted with a solution containing a 1:1 ratio of $CH_2Cl_2$ to MeOH. Two additional drops of concentrated sulfuric acid were then added to the reaction mixture. After about 3 hours, TLC analysis still mainly showed presence of compound (6). LC-MS showed a minor amount, about 7%, of a product with the correct mass for compound (11), but still about 85% of compound (6). The reaction mixture was then treated with 0.5 mL of $H_2O$ and three drops of concentrated sulfuric acid. After stirring at room temperature for about 21 hours, HPLC analysis showed no change in the reaction mixture. The reaction mixture, which had a turbid appearance at this stage, was treated with two more drops of concentrated sulfuric acid, warmed with a heat gun, and became homogenous. The mixture was then allowed to cool to room temperature. HPLC analysis was performed again and still mainly showed compound (6). The reaction mixture was then warmed with a heating mantle. After stirring at 70 °C for 4 hours, HPLC analysis indicated that all of compound (6) had been consumed and the presence of one major, more polar product. LC-MS showed a major product with the correct mass for compound (11). The reaction mixture was then concentrated under a stream of $N_2$, and the oil was taken up in warm $CH_3CN$ and blown multiple times to dryness. The residual dark yellow oil was dissolved in warm isopropanol and the solution placed in a refrigerator.

No crystals or solid had formed after 3 days in the refrigerator. The solvent was then removed with a stream of $N_2$ and the residue was dissolved in $CH_2Cl_2$ with a minor amount of methanol. The residue was then chromatographed by flash chromatography using a Teledyne-Isco CombiFlash Companion® (Isco, Inc., Lincoln, Nebraska) flash chromatography system equipped with a 12 g RediSep silica gel column (Isco, Inc.). The chromatography was performed with a flow rate of 30 mL/minute, detection at 254 nm, and dichloromethane and dichloromethane with 10% methanol were used as solvents. The following stepwise gradient was used: 100% dichloromethane for 2 minutes; 80% dichloromethane/20% dichloromethane/10% methanol for 2 minutes; 60% dichloromethane/40% dichloromethane/10% methanol for 2 minutes; 40% dichloromethane/60% dichloromethane/10% methanol for 2 minutes; 20% dichloromethane/80% dichloromethane/10% methanol for 2 minutes; and 100% dichloromethane/10% methanol for 4 minutes. Fractions containing the major product were combined and concentrated in vacuo to give 94 mg of a tan foam. Found: $^1H$ NMR (300 MHz, DMSO-d6) δ 8.53 (dd, *J* = 4.1, 1.4 Hz, 1H), 7.97 (dd, *J* = 8.4, 5.5 Hz, 1H), 7.61 (dd, *J* = 8.5, 4.3 Hz, 1H), 6.36 (s, 1H), 6.11 (s, 1H), 4.99 (dq, *J* = 13.9, 7.0 Hz, 1H), 3.22 - 3.08 (m, 3H), 1.73-1.67 (m, 3H). ESI MS (m/z) 312 [M+H]+.

**Examples 11-14:**

Compounds (18), (19), (20 - Reference) and (21) of Examples 11-14, respectively, are shown in Table 3 below. Compounds (14), (15), (16) and (17) (also shown in Table 3 below) were oxidized utilizing processes similar to those described above in connection with Examples 8 and 9 to provide compounds (18), (19), (20 - Reference) and (21), respectively.

TABLE 3

| Starting Compounds | Oxidized Compounds |
|---|---|
| 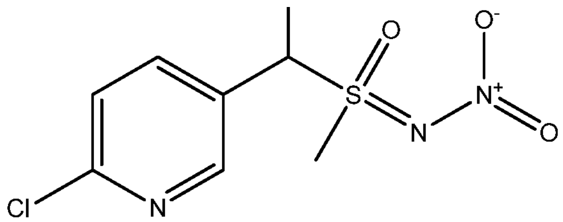 (14) | 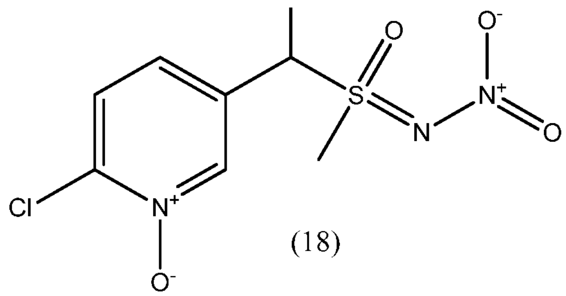 (18) |
| | Found: $^1$H NMR (400 MHz, DMSO-d6, mixture of diasteriomers) 8.63-8.61 (m, 1H), 7.93-7.90 (m, 1H), 7.53-7.41 (m, 1H), 5.37-5.32 (m, 1H), 3.66 (s, 1.28H), 3.63 (s, 1.74H), 1.82-1.79 (m, 3H). ESI MS (*m*/*z*) 280 [M+H]+, 278 [M-H]-. |
| 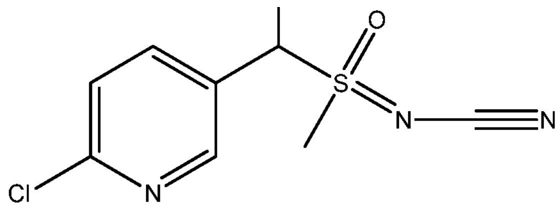 (15) | 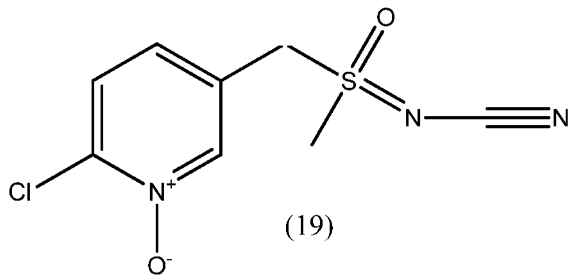 (19) |
| | Found: $^1$H NMR (400 MHz, DMSO-d6, mixture of diasteriomers) δ 8.59 (d, J = 1.9 Hz, 1H), 7.90 (d, J = 8.5 Hz, 1H), 7.48 (dt, J = 8.6, 1.9 Hz, 1H), 5.16 (q, J = 7.1 Hz, 1H), 3.46 (s, 1.1H), 3.44 (s, 1.9H), 1.80 (d, J = 7.1 Hz, 3H). ESI MS (*m*/*z*)261 [M+H]+, 258 [M-H]-. |
| 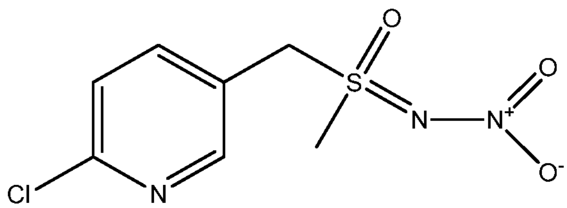 (16) (reference) | 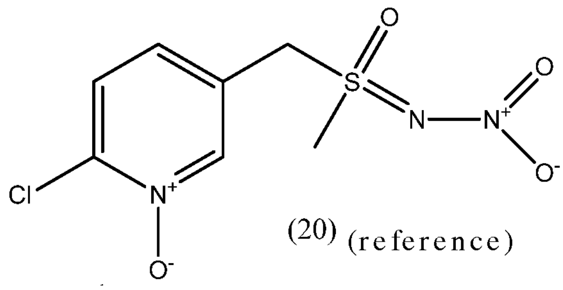 (20) (reference) |
| | Found: $^1$H NMR (400 MHz, DMSO-d6) δ 8.54 (d, *J* = 1.8 Hz, 1H), 7.91 (d, *J* = 8.5 Hz, 1H), 7.41 (dd, *J* = 8.5, 1.8 Hz, 1H), 5.23 (d, *J* = 2.4 Hz, 2H), 3.62 (s, 3H). ESI MS (*m*/*z*) 268 $[M+H]^+$, 264 $[M-H]^-$. |

(continued)

| Starting Compounds | Oxidized Compounds |
|---|---|
| 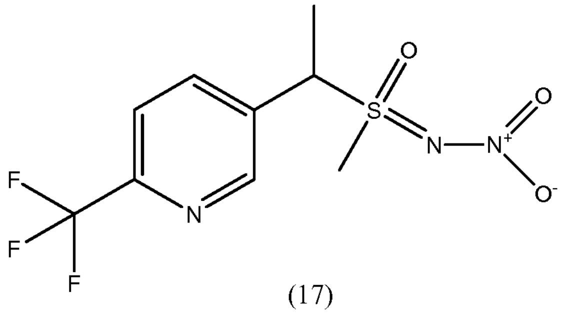 (17) | 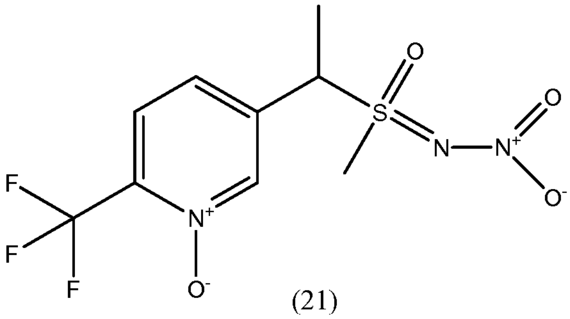 (21) |
| | Found: $^1$H NMR (400 MHz, DMSO-d6, mixture of diasteriomers) 8.63-8.61 (m, 1H), 8.08-8.05 (m, 1H), 7.68-7.63 (m, 1H), 5.43-5.39(m, 1H), 3.71 (s, 1.4H), 3.67 (s, 1.6H), 1.99-1.82 (m 3H). ESI MS (*m*/*z*) 314 $[M+H]^+$, 312 $[M-H]^-$. |

**Examples 15-16:**

Compounds (22) and (23) of Examples 15-16, respectively, are shown in Table 4 below. Compounds (9) and (19) (also shown in Table 4 below) were acid hydrolyzed utilizing a process similar to that described above in connection with Example 10 to provide compounds (22) and (23), respectively.

TABLE 4

| Starting Compounds | Acid Hydrolyzed (Reference) |
|---|---|
| 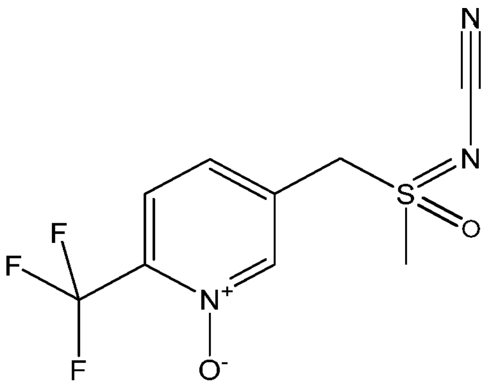 (9) | 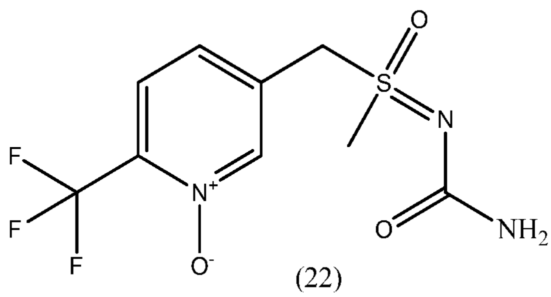 (22) |
| | Found: $^1$H NMR (300 MHz, DMSO-d6) δ 8.48 (d, *J* = 1.3 Hz, 1H), 8.00 (d, *J* = 8.3 Hz, 1H), 7.54 (dd, *J* = 8.2, 1.4 Hz, 1H), 6.40 (s, 1H), 6.18 (s, 1H), 4.93 (s, 2H), 3.14 (s, 3H). ESI MS (m/z) 298 $[M+H]^+$. |
| 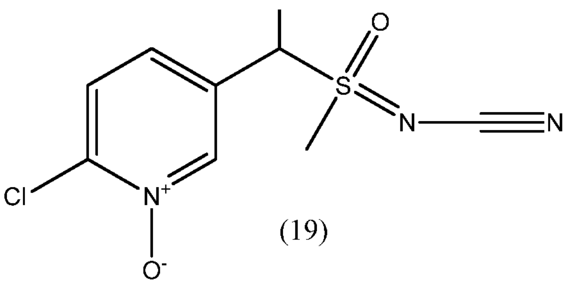 (19) | 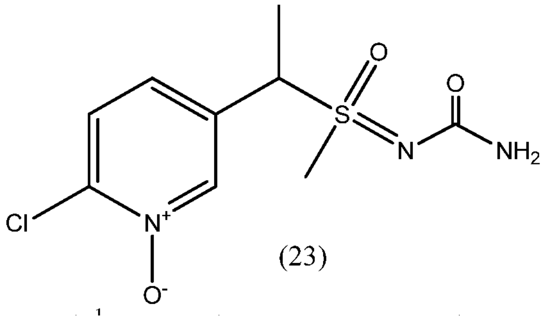 (23) |

(continued)

| Starting Compounds | Acid Hydrolyzed (Reference) |
|---|---|
| | Found: $^1H$ NMR (400 MHz, DMSO-d6, mixture of diasteriomers) 8.56-8.55 (m, 1H), 7.87-7.83 (m,1H), 7.50-7.46 (m, 1H), 6.37 (bs, 1H), 6.08 (bs, 1H), 4.96-4.87 (m, 1H), 3.16 (s, 1.4H), 3.15 (s, 1.6H)1.71-1.68 (m, 3H). ESI MS (*m*/*z*) 278 $[M+H]^+$. |

**Example 17: Insecticidal Testing for Green Peach Aphid (GPA):**

GPA is the most significant aphid pest of peach trees, causing decreased growth, shriveling of the leaves, and the death of various tissues. It is also hazardous because it acts as a vector for the transport of plant viruses, such as potato virus Y and potato leafroll virus to members of the nightshade/potato family *Solanaceae,* and various mosaic viruses to many other food crops. GPA attacks such plants as broccoli, burdock, cabbage, carrot, cauliflower, daikon, eggplant, green beans, lettuce, macadamia, papaya, peppers, sweet potatoes, tomatoes, watercress, and zucchini, among other plants. GPA also attacks many ornamental crops such as carnation, chrysanthemum, flowering white cabbage, poinsettia, and roses. GPA has developed resistance to many pesticides.

Certain compounds disclosed in this document were tested against GPA using procedures described as follows. Cabbage seedlings grown in 3-inch pots, with 2-3 small (3-5 cm) true leaves, were used as test substrate. The seedlings were infested with 20-50 GPA (wingless adult and nymph stages) one day prior to chemical application. Four pots with individual seedlings were used for each treatment. Test compounds (2 mg) were dissolved in 2 mL of acetone/methanol (1:1) solvent, forming stock solutions of 1000 ppm test compound. The stock solutions were diluted 5X with 0.025% Tween® 20 (Sigma-Aldrich, Inc., St. Louis, MO) in $H_2O$ to obtain the solution at 200 ppm test compound. A hand-held aspirator-type sprayer was used for spraying a solution to both sides of cabbage leaves until runoff. Reference plants (solvent check) were sprayed with the diluent only containing 20% by volume of acetone/methanol (1:1) solvent. Treated plants were held in a holding room for three days at approximately 25 °C and ambient relative humidity (RH) prior to grading. Evaluation was conducted by counting the number of live aphids per plant under a microscope. Percent Control was measured by using Abbott's correction formula (W.S. Abbott, "A Method of Computing the Effectiveness of an Insecticide" J. Econ. Entomol. 18 (1925), pp.265-267) as follows:

$$\text{Corrected \% Control} = 100 * (X - Y) / X$$

where

X = No. of live aphids on solvent check plants and
Y = No. of live aphids on treated plants

The results provided in Table 6 below are based on the qualitative ratings set forth in Table 5.

TABLE 5

| GPA Rating Table | |
|---|---|
| **% Control (or Mortality)** | **Rating** |
| 80-100 | A |
| More than 0 - Less than 80 | B |
| Not Tested | C |
| No activity noticed in this bioassay | D |

TABLE 6

| Compound | GPA Control @ 200 ppm |
|---|---|
| (5) | **A** |

(continued)

| Compound | GPA Control @ 200 ppm |
|---|---|
| (6) | A |
| (9) | A |
| (10) | A |
| (11)Ref. | C |
| (18) | B |
| (19) | A |
| (20)Ref. | D |
| (21) | A |
| (22)Ref. | D |
| (23)Ref. | D |

Insecticide Utility

The compounds disclosed herein are useful for the control of invertebrates including insects. Accordingly, one embodiment disclosed herein is directed to a method for inhibiting an insect which comprises applying an insect-inhibiting amount of a compound according to formula (I) to a locus of the insect, to the area to be protected, or directly on the insect to be controlled. The compounds of disclosed herein may also be used to control other invertebrate pests such as mites and nematodes.

The "locus" of insects or other pests is a term used herein to refer to the environment in which the insects or other pests live or where their eggs are present, including the air surrounding them, the food they eat, or objects which they contact. For example, insects which eat, damage or contact edible, commodity, ornamental, turf or pasture plants can be controlled by applying the active compounds to the seed of the plant before planting, to the seedling, or cutting which is planted, the leaves, stems, fruits, grain, and/or roots, or to the soil or other growth medium before or after the crop is planted. Protection of these plants against virus, fungus or bacterium diseases may also be achieved indirectly through controlling sap-feeding pests such as whitefly, plant hopper, aphid and spider mite. Such plants include those which are bred through conventional approaches and which are genetically modified using modern biotechnology to gain insect-resistant, herbicide-resistant, nutrition-enhancement, and/or any other beneficial traits.

It is contemplated that the compounds disclosed herein might also be useful to protect textiles, paper, stored grain, seeds and other foodstuffs, houses and other buildings which may be occupied by humans and/or companion, farm, ranch, zoo, or other animals, by applying an active compound to or near such objects. Domesticated animals, buildings or human beings might be protected with the compounds by controlling invertebrate and/or nematode pests that are parasitic or are capable of transmitting infectious diseases. Such pests include, for example, chiggers, ticks, lice, mosquitoes, flies, fleas and heartworms. Nonagronomic applications also include invertebrate pest control in forests, in yards, along road sides and railroad right of way.

The term "inhibiting an insect" refers to a decrease in the numbers of living insects, or a decrease in the number of viable insect eggs. The extent of reduction accomplished by a compound depends, of course, upon the application rate of the compound, the particular compound used, and the target insect species. At least an inactivating amount should be used. The term "insect-inactivating amount" is used to describe the amount which is sufficient to cause a measurable reduction in the treated insect population. Generally, an amount in the range from about 1 to about 1000 ppm by weight of active compound is used. For example, insects or other pests which can be inhibited include, but are not limited to:

Lepidoptera--*Heliothis* spp., *Helicoverpa* spp., *Spodoptera* spp., *Mythimna unipuncta, Agrotis ipsilon, Earias* spp., *Euxoa auxiliaris, Trichoplusia ni, Anticarsia gemmatalis, Rachiplusia nu, Plutella xylostella, Chilo* spp., *Scirpophaga incertulas, Sesamia inferens, Cnaphalocrocis medinalis, Ostrinia nubilalis, Cydia pomonella, Carposina niponensis, Adoxophyes orana, Archips argyrospilus, Pandemis heparana, Epinotia aporema, Eupoecilia ambiguella, Lobesia botrana, Polychrosis viteana, Pectinophora gossypiella, Pieris rapae, Phyllonorycter* spp., *Leucoptera malifoliella, Phyllocnisitis citrella;*

Coleoptera--*Diabrotica* spp., *Leptinotarsa decemlineata, Oulema oryzae, Anthonomus grandis, Lissorhoptrus oryzophilus, Agriotes* spp., *Melanotus communis, Popillia japonica, Cyclocephala* spp., *Tribolium* spp.;

Homoptera--*Aphis* spp., *Myzus persicae, Rhopalosiphum* spp., *Dysaphis plantaginea, Toxoptera* spp., *Macrosiphum euphorbiae, Aulacorthum solani, Sitobion avenae, Metopolophium dirhodum, Schizaphis graminum, Brachycolus noxius, Nephotettix* spp., *Nilaparvata lugens, Sogatella furcifera, Laodelphax striatellus, Bemisia tabaci, Trialeurodes vaporariorum, Aleurodes proletella, Aleurothrixus floccosus, Quadraspidiotus perniciosus, Unaspis yanonensis, Ceroplastes rubens, Aonidiella aurantii;*

Hemiptera--*Lygus* spp., *Eurygaster maura, Nezara viridula, Piezodorus guildingi, Leptocorisa varicornis, Cimex lectularius, Cimex hemipterus;*

Thysanoptera--*Frankliniella* spp., *Thrips* spp., *Scirtothrips dorsalis;*

Isoptera--*Reticulitermes flavipes, Coptotermes formosanus, Reticulitermes virginicus, Heterotermes aureus, Reticulitermes hesperus, Coptotermes frenchii, Shedorhinotermes* spp., *Reticulitermes santonensis, Reticulitermes grassei, Reticulitermes banyulensis, Reticulitermes speratus, Reticulitermes hageni, Reticulitermes tibialis, Zootermopsis* spp., *Incisitermes* spp., *Marginitermes* spp., *Macrotermes* spp., *Microcerotermes* spp., *Microtermes* spp.;

Diptera--*Liriomyza* spp., *Musca domestica, Aedes* spp., *Culex* spp., *Anopheles* spp., *Fannia* spp., *Stomoxys* spp.;

*Hymenoptera--Iridomyrmex humilis, Solenopsis* spp., *Monomorium pharaonis, Atta* spp., *Pogonomyrmex* spp., *Camponotus* spp., *Monomorium* spp., *Tapinoma sessile, Tetramorium* spp., *Xylocapa* spp., *Vespula* spp., *Polistes* spp.;

Mallophaga (chewing lice);

Anoplura (sucking lice)--*Pthirus pubis, Pediculus* spp.;

Orthoptera (grasshoppers, crickets)--*Melanoplus* spp., *Locusta migratoria, Schistocerca gregaria, Gryllotalpidae* (mole crickets);

Blattoidea (cockroaches)--*Blatta orientalis, Blattella germanica, Periplaneta americana, Supella longipalpa, Periplaneta australasiae, Periplaneta brunnea, Parcoblatta pennsylvanica, Periplaneta fuliginosa, Pycnoscelus surinamensis;*

Siphonaptera--*Ctenophalides* spp., *Pulex irritans;*

Acari--*Tetranychus* spp., *Panonychus* spp., *Eotetranychus carpini, Phyllocoptruta oleivora, Aculus pelekassi, Brevipalpus phoencis, Boophilus* spp., *Dermacentor variabilis, Rhipicephalus sanguineus, Amblyomma americanum, Ixodes* spp., *Notoedres cati, Sarcoptes scabiei, Dermatophagoides* spp.; and

Nematoda--Dirofilaria immitis, Meloidogyne spp., *Heterodera* spp., *Hoplolaimus columbus, Belonolaimus* spp., *Pratylenchus* spp., *Rotylenchus reniformis, Criconemella ornata, Ditylenchus* spp., *Aphelenchoides besseyi, Hirschmanniella* spp.

## Compositions

The compounds disclosed herein may be applied in the form of compositions which include a compound disclosed herein and a phytologically-acceptable inert carrier. Control of the pests is achieved by applying compounds disclosed herein in forms of sprays, topical treatment, gels, seed coatings, microcapsulations, systemic uptake, baits, eartags, boluses, foggers, fumigants aerosols, dusts and many others. The compositions are either concentrated solid or liquid formulations which are dispersed in water for application, or are dust or granular formulations which are applied without further treatment. The compositions are prepared according to procedures and formulae which are conventional in the agricultural chemical art, but which are novel and important because of the presence therein of the compounds disclosed herein. Some description of the formulation of the compositions will be given, however, to assure that agricultural chemists can readily prepare any desired composition.

The dispersions in which the compounds are applied are most often aqueous suspensions or emulsions prepared from concentrated formulations of the compounds. Such water-soluble, water-suspendable or emulsifiable formulations are either solids, usually known as wettable powders, or liquids usually known as emulsifiable concentrates or aqueous suspensions. Wettable powders, which may be compacted to form water dispersible granules, comprise an intimate mixture of the active compound, an inert carrier, and surfactants. The concentration of the active compound is usually from about 10% to about 90% by weight. The inert carrier is usually chosen from among the attapulgite clays, the montmorillonite clays, the diatomaceous earths, or the purified silicates. Effective surfactants, comprising from about 0.5% to about 10% of the wettable powder, are found among the sulfonated lignins, the condensed naphthalenesulfonates, the naphthalenesulfonates, the alkylbenzenesulfonates, the alkyl sulfates, and nonionic surfactants such as ethylene oxide adducts of alkyl phenols.

Emulsifiable concentrates of the compounds comprise a convenient concentration of a compound, such as from about 50 to about 500 grams per liter of liquid, equivalent to about 10% to about 50%, dissolved in an inert carrier which is either a water miscible solvent or a mixture of water-immiscible organic solvent and emulsifiers. Useful organic solvents include aromatics, especially the xylenes, and the petroleum fractions, especially the high-boiling naphthalenic and olefinic portions of petroleum such as heavy aromatic naphtha. Other organic solvents may also be used, such as the terpenic solvents including rosin derivatives, aliphatic ketones such as cyclohexanone, and complex alcohols such as 2-ethoxyethanol. Suitable emulsifiers for emulsifiable concentrates are chosen from conventional anionic and/or

nonionic surfactants, such as those discussed above.

Aqueous suspensions comprise suspensions of water-insoluble compounds dispersed in an aqueous vehicle at a concentration in the range from about 5% to about 50% by weight. Suspensions are prepared by finely grinding the compound, and vigorously mixing it into a vehicle comprised of water and surfactants chosen from the same types discussed above. Inert ingredients, such as inorganic salts and synthetic or natural gums, may also be added, to increase the density and viscosity of the aqueous vehicle. It is often most effective to grind and mix the compound at the same time by preparing the aqueous mixture, and homogenizing it in an implement such as a sand mill, ball mill, or piston-type homogenizer.

The compounds may also be applied as granular compositions, which are particularly useful for applications to the soil. Granular compositions usually contain from about 0.5% to about 10% by weight of the compound, dispersed in an inert carrier which consists entirely or in large part of clay or a similar inexpensive substance. Such compositions are usually prepared by dissolving the compound in a suitable solvent and applying it to a granular carrier which has been pre-formed to the appropriate particle size, in the range of from about 0.5 to 3 mm. Such compositions may also be formulated by making a dough or paste of the carrier and compound and crushing and drying to obtain the desired granular particle size. Dusts containing the compounds are prepared simply by intimately mixing the compound in powdered form with a suitable dusty agricultural carrier, such as kaolin clay, ground volcanic rock, and the like. Dusts can suitably contain from about 1% to about 10% of the compound. It is equally practical, when desirable for any reason, to apply the compound in the form of a solution in an appropriate organic solvent, usually a bland petroleum oil, such as the spray oils, which are widely used in agricultural chemistry.

Insecticides and acaricides are generally applied in the form of a dispersion of the active ingredient in a liquid carrier. It is conventional to refer to application rates in terms of the concentration of active ingredient in the carrier. The most widely used carrier is water.

The compounds of the disclosed herein can also be applied in the form of an aerosol composition. In such compositions the active compound is dissolved or dispersed in an inert carrier, which is a pressure-generating propellant mixture. The aerosol composition is packaged in a container from which the mixture is dispensed through an atomizing valve. Propellant mixtures comprise either low-boiling halocarbons, which may be mixed with organic solvents, or aqueous suspensions pressurized with inert gases or gaseous hydrocarbons.

The actual amount of compound to be applied to loci of insects and mites is not critical and can readily be determined by those skilled in the art in view of the examples above. In general, concentrations from 10 ppm to 5000 ppm by weight of compound are expected to provide good control. With many of the compounds, concentrations from 100 to 1500 ppm will suffice.

The locus to which a compound is applied can be any locus inhabited by an insect or mite, for example, vegetable crops, fruit and nut trees, grape vines, ornamental plants, domesticated animals, the interior or exterior surfaces of buildings, and the soil around buildings.

Because of the unique ability of insect eggs to resist toxicant action, repeated applications may be desirable to control newly emerged larvae, as is true of other known insecticides and acaricides.

Systemic movement of compounds disclosed herein in plants may be utilized to control pests on one portion of the plant by applying the compounds to a different portion of it. For example, control of foliar-feeding insects can be controlled by drip irrigation or furrow application, or by treating the seed before planting. Seed treatment can be applied to all types of seeds, including those from which plants genetically transformed to express specialized traits will germinate. Representative examples include those expressing proteins toxic to invertebrate pests, such as *Bacillus thuringiensis* or other insecticidal proteins, those expressing herbicide resistance, such as "Roundup Ready®" seed, or those with "stacked" foreign genes expressing insecticidal proteins, herbicide resistance, nutrition-enhancement and/or any other beneficial traits.

An insecticidal bait composition consisting of compounds disclosed herein and attractants and/or feeding stimulants may be used to increase efficacy of the insecticides against insect pest in a device such as trap, bait station, and the like. The bait composition is usually a solid, semi-solid (including gel) or liquid bait matrix including the stimulants and one or more non-microencapsulated or microencapsulated insecticides in an amount effective to act as kill agents.

Compounds according to formula (I) may also be applied in conjunction with one or more other insecticides or fungicides or herbicides to obtain control of a wider variety of pests diseases and weeds. When used in conjunction with other insecticides or fungicides or herbicides, the compounds according to formula (I) can be formulated with the other insecticides or fungicides or herbicide, tank mixed with the other insecticides or fungicides or herbicides, or applied sequentially with the other insecticides or fungicides or herbicides.

Some of the insecticides that can be employed beneficially in combination with the compounds disclosed herein include: antibiotic insecticides such as allosamidin and thuringiensin; macrocyclic lactone insecticides such as spinosad, spinetoram, and other spinosyns including the 21-butenyl spinosyns and their derivatives; avermectin insecticides such as abamectin, doramectin, emamectin, eprinomectin, ivermectin and selamectin; milbemycin insecticides such as lepimectin, milbemectin, milbemycin oxime and moxidectin; arsenical insecticides such as calcium arsenate, copper acetoarsenite, copper arsenate, lead arsenate, potassium arsenite and sodium arsenite; biological insecticides such as *Bacillus popilliae, B. sphaericius, B. thurinigiensis* subsp. *aizawai, B. thuringiensis* subsp. *kurstaki, B. thuriugiensis* subsp. *tenebrionis, Beauveria bassiana, Cydia pomonella* granulosis virus, Douglas fir tussock moth NPV, gypsy moth NPV, *Helicoverpa zea* NPV, Indian meal moth granulosis virus, *Metarhizium anisopliae, Nosema locustae, Paecilomyces fumosoroseus, P. lilacinus, Photorhabdus luminescens, Spodoptera exigua* NPV, trypsin modulating oostatic factor, *Xenorhabdus nematophilus,* and *X. bovienii,* plant incorporated protectant insecticides such as Cry1Ab, Cry1Ac, Cry1F, Cry1A.105, Cry2Ab2, Cry3A, mir Cry3A, Cry3Bb1, Cry34, Cry35, and VIP3A; botanical insecticides such as anabasine, azadirachtin, d-limonene, nicotine, pyrethrins, cinerins, cinerin I, cinerin II, jasmolin I, jasmolin II, pyrethrin I, pyrethrin II, quassia, rotenone, ryania and sabadilla; carbamate insecticides such as bendiocarb and carbaryl; benzofuranyl methylcarbamate insecticides such as benfuracarb, carbofuran, carbosulfan, decarbofuran and furathiocarb; dimethylcarbamate insecticides dimitan, dimetilan, hyquincarb and pirimicarb; oxime carbamate insecticides such as alanycarb, aldicarb, aldoxycarb, butocarboxim, butoxycarboxim, methomyl, nitrilacarb, oxamyl, tazimcarb, thiocarboxime, thiodicarb and thiofanox; phenyl methylcarbamate insecticides such as allyxycarb, aminocarb, bufencarb, butacarb, carbanolate, cloethocarb, dicresyl, dioxacarb, EMPC, ethiofencarb, fenethacarb, fenobucarb, isoprocarb, methiocarb, metolcarb, mexacarbate, promacyl, promecarb, propoxur, trimethacarb, XMC and xylylcarb; dinitrophenol insecticides such as dinex, dinoprop, dinosam and DNOC; fluorine insecticides such as barium hexafluorosilicate, cryolite, sodium fluoride, sodium hexafluorosilicate and sulfluramid; formamidine insecticides such as amitraz, chlordimeform, formetanate and formparanate; fumigant insecticides such as acrylonitrile, carbon disulfide, carbon tetrachloride, chloroform, chloropicrin, para-dichlorobenzene, 1,2-dichloropropane, ethyl formate, ethylene dibromide, ethylene dichloride, ethylene oxide, hydrogen cyanide, iodomethane, methyl bromide, methylchloroform, methylene chloride, naphthalene, phosphine, sulfuryl fluoride and tetrachloroethane; inorganic insecticides such as borax, calcium polysulfide, copper oleate, mercurous chloride, potassium thiocyanate and sodium thiocyanate; chitin synthesis inhibitors such as bistrifluron, buprofezin, chlorfluazuron, cyromazine, diflubenzuron, flucycloxuron, flufenoxuron, hexaflumuron, lufenuron, novaluron, noviflumuron, penfluron, teflubenzuron and triflumuron; juvenile hormone mimics such as epofenonane, fenoxycarb, hydroprene, kinoprene, methoprene, pyriproxyfen and triprene; juvenile hormones such as juvenile hormone I, juvenile hormone II and juvenile hormone III; moulting hormone agonists such as chromafenozide, halofenozide, methoxyfenozide and tebufenozide; moulting hormones such as $\alpha$-ecdysone and ecdysterone; moulting inhibitors such as diofenolan; precocenes such as precocene I, precocene II and precocene III; unclassified insect growth regulators such as dicyclanil; nereistoxin analogue insecticides such as bensultap, cartap, thiocyclam and thiosultap; nicotinoid insecticides such as flonicamid; nitroguanidine insecticides such as clothianidin, dinotefuran, imidacloprid and thiamethoxam; nitromethylene insecticides such as nitenpyram and nithiazine; pyridylmethylamine insecticides such as acetamiprid, imidacloprid, nitenpyram and thiacloprid; organochlorine insecticides such as bromo-DDT, camphechlor, DDT, pp'-DDT, ethyl-DDD, HCH, gamma-HCH, lindane, methoxychlor, pentachlorophenol and TDE; cyclodiene insecticides such as aldrin, bromocyclen, chlorbicyclen, chlordane, chlordecone, dieldrin, dilor, endosulfan, endrin, HEOD, heptachlor, HHDN, isobenzan, isodrin, kelevan and mirex; organophosphate insecticides such as bromfenvinfos, chlorfenvinphos, crotoxyphos, dichlorvos, dicrotophos, dimethylvinphos, fospirate, heptenophos, methocrotophos, mevinphos, monocrotophos, naled, naftalofos, phosphamidon, propaphos, TEPP and tetrachlorvinphos; organothiophosphate insecticides such as dioxabenzofos, fosmethilan and phenthoate; aliphatic organothiophosphate insecticides such as acethion, amiton, cadusafos, chlorethoxyfos, chlormephos, demephion, demephion-O, demephion-S, demeton, demeton-O, demeton-S, demeton-methyl, demeton-O-methyl, demeton-S-methyl, demeton-S-methylsulphon, disulfoton, ethion, ethoprophos, PSP, isothioate, malathion, methacrifos, oxydemeton-methyl, oxydeprofos, oxydisulfoton, phorate, sulfotep, terbufos and thiometon; aliphatic amide organothiophosphate insecticides such as amidithion, cyanthoate, dimethoate, ethoate-methyl, formothion, mecarbam, omethoate, prothoate, sophamide and vamidothion; oxime organothiophosphate insecticides such as chlorphoxim, phoxim and phoxim-methyl; heterocyclic organothiophosphate insecticides such as azamethiphos, coumaphos, coumithoate, dioxathion, endothion, menazon, morphothion, phosalone, pyraclofos, pyridaphenthion and quinothion; benzothiopyran organothiophosphate insecticides such as dithicrofos and thicrofos; benzotriazine organothiophosphate insecticides such as azinphos-ethyl and azinphos-methyl; isoindole organothiophosphate insecticides such as dialifos and phosmet; isoxazole organothiophosphate insecticides such as isoxathion and zolaprofos; pyrazolopyrimidine organothiophosphate insecticides such as chlorprazophos and pyrazophos; pyridine organothiophosphate insecticides such as chlorpyrifos and chlorpyrifos-methyl; pyrimidine organothiophosphate insecticides such as butathiofos, diazinon, etrimfos, lirimfos, pirimiphos-ethyl, pirimiphos-methyl, primidophos, pyrimitate and tebupirimfos; quinoxaline organothiophosphate insecticides such as quinalphos and quinalphos-methyl; thiadiazole organothiophosphate insecticides such as athidathion, lythidathion, methidathion and prothidathion; triazole organothiophosphate insecticides such as isazofos and triazophos; phenyl organothiophosphate insecticides such as azothoate, bromophos, bromophos-ethyl, carbophenothion, chlorthiophos, cyanophos, cythioate, dicapthon, dichlofenthion, etaphos, famphur, fenchlorphos, fenitrothion fensulfothion, fenthion, fenthion-ethyl, heterophos, jodfenphos, mesulfenfos, parathion, parathion-methyl, phenkapton, phosnichlor, profenofos, prothiofos, sulprofos, temnephos, trichlormetaphos-3 and trifenofos; phosphloiate insecticides such as butonate and trichlorfon; phosphonothioate insecticides such as mecarphon; phenyl ethylphosplionotliioate insecticides such as fonofos and trichloronat; phenyl phenylphosphonothioate insecticides such as cyanofenphos, EPN and leptophos; phosphoramidate insecticides such as crufomate, fenamiphos, fosthietan, mephosfolan,phosfolan and pirimetaphos; phosphoramidothioate insecticides such as acephate, isocarbophos, isofenphos, methamidophos and propetamphos; phosphorodiamide insecticides such as dimefox, mazidox, mipafox and schradan; oxadiazine insecticides such as indoxacarb; phthalimide insecticides such as dialifos, phosmet and tetramethrin; pyrazole insecticides such as acetoprole, ethiprole, fipronil, pyrafluprole, pyriprole, tebufenpyrad, tolfenpyrad and vaniliprole; pyrethroid ester insecticides such as acrinathrin, allethrin, bioallethrin, barthrin, bifenthrin, bioethanomethrin, cyclethrin, cycloprothrin, cyfluthrin, beta-cyfluthrin, cyhalothrin, gamma-cyhalothrin, lambda-cyhalothrin, cypermethrin, alpha-cypermethrin, beta-cypermethrin, theta-cypermethrin, zeta-cypermethrin, cyphenothrin, deltamethrin, dimefluthrin, dimethrin, empenthrin, fenfluthrin, fenpirithrin, fenpropathrin, fenvalerate, esfenvalerate, flucythrinate, fluvalinate, tau-fluvalinate, furethrin, imiprothrin, metofluthrin, permethrin, biopermethrin, transpermethrin, phenothrin, prallethrin, profluthrin, pyresmethrin, resmethrin, bioresmethrin, cismethrin, tefluthrin, terallethrin, tetramethrin, tralomethrin and transfluthrin; pyrethroid ether insecticides such as etofenprox, flufenprox, halfenprox, protrifenbute and silafluofen; pyrimidinamine insecticides such as flufenerim and pyrimidifen; pyrrole insecticides such as chlorfenapyr; tetronic acid insecticides such as spirodiclofen, spiromesifen and spirotetramat; thiourea insecticides such as diafenthiuron; urea insecticides such as flucofuron and sulcofuron; and unclassified insecticides such as AKD-3088, closantel, crotamiton, cyflumetofen, E2Y45, EXD, fenazaflor, fenazaquin, fenoxacrim, fenpyroximate, FKI-1033, flubendiamide, HGW86, hydramethylnon, IKI-2002, isoprothiolane, malonoben, metaflumizone, metoxadiazone, nifluridide, NNI-9850, NNI-0101, pymetrozine, pyridaben, pyridalyl, Qcide, rafoxanide, rynaxypyr, SYJ-159, triarathene and triazamate and any combinations thereof.

Some of the fungicides that can be employed beneficially in combination with the compounds disclosed herein include: 2-(thiocyanatomethylthio)-benzothiazole, 2-phenylphenol, 8-hydroxyquinoline sulfate, Ampelomyces, quisqualis, azaconazole, azoxystrobin, *Bacillus subtilis,* benalaxyl, benomyl, benthiavalicarb-isopropyl, benzylaminobenzenesulfonate (BABS) salt, bicarbonates, biphenyl, bismerthiazol, bitertanol, blasticidin-S, borax, Bordeaux mixture, boscalid, bromuconazole, bupirimate, calcium polysulfide, captafol, captan, carbendazim, carboxin, carpropamid, carvone, chloroneb, chlorothalonil, chlozolinate, *Coniothyrium minitans,* copper hydroxide, copper octanoate, copper oxychloride, copper sulfate, copper sulfate (tribasic), cuprous oxide, cyazofamid, cyflufenamid, cymoxanil, cyproconazole, cyprodinil, dazomet, debacarb, diammonium ethylenebis-(dithiocarbamate), dichlofluanid, dichlorophen, diclocymet, diclomezine, dichloran, diethofencarb, difenoconazole, difenzoquat ion, diflumetorim, dimethomorph, dimoxystrobin, diniconazole, diniconazole-M,dinobuton, dinocap, diphenylamine, dithianon, dodemorph, dodemorph acetate, dodine, dodine free base, edifenphos, epoxiconazole, ethaboxam, ethoxyquin, etridiazole, famoxadone, fenamidone, fenarimol, fenbuconazole, fenfuram, fenhexamid, fenoxanil, fenpiclonil, fenpropidin, fenpropimorph, fentin, fentin acetate, fentin hydroxide, ferbam, ferimzone, fluazinam, fludioxonil, flumorph, fluopicolide, fluoroimide, fluoxastrobin, fluquinconazole, flusilazole, flusulfamide, flutolanil, flutriafol, folpet, formaldehyde, fosetyl, fosetyl-aluminium, fuberidazole, furalaxyl, furametpyr, guazatine, guazatine acetates, GY-81, hexachlorobenzene, hexaconazole, hymexazol, imazalil, imazalil sulfate, imibenconazole, iminoctadine, iminoctadine triacetate, iminoctadine tris(albesilate), ipconazole, iprobenfos, iprodione, iprovalicarb, isoprothiolane, kasugamycin, kasugamycin hydrochloride hydrate, kresoxim-methyl, mancopper, mancozeb, maneb, mepanipyrim, mepronil, mercuric chloride, mercuric oxide, mercurous chloride, metalaxyl, mefenoxam, metalaxyl-M, metam, metam-ammonium, metam-potassium, metam-sodium, metconazole, methasulfocarb, methyl iodide, methyl isothiocyanate, metiram, metominostrobin, metrafenone, mildiomycin, myclobutanil, nabam, nitrothal-isopropyl, nuarimol, octhilinone, ofurace, oleic acid (fatty acids), orysastrobin, oxadixyl, oxine-copper, oxpoconazole fumarate, oxycarboxin, pefurazoate, penconazole, pencycuron, pentachlorophenol, pentachlorophenyl laurate, penthiopyrad, phenylmercury acetate, phosphonic acid, phthalide, picoxystrobin, polyoxin B, polyoxins, polyoxorim, potassium bicarbonate, potassium hydroxyquinoline sulfate, probenazole, prochloraz, procymidone, propamocarb, propamocarb hydrochloride, propiconazole, propineb, proquinazid, prothioconazole, pyraclostrobin, pyrazophos, pyributicarb, pyrifenox, pyrimethanil, pyroquilon, quinoclamine, quinoxyfen, quintozene, Reynoutria sachalinensis extract, silthiofam, simeconazole, sodium 2-phenylphenoxide, sodium bicarbonate, sodium pentachlorophenoxide, spiroxamine, sulfur, SYP-Z071, tar oils, tebuconazole, tecnazene, tetraconazole, thiabendazole, thifluzamide, thiophanate-methyl, thiram, tiadinil, tolclofos-methyl, tolylfluanid, triadimefon, triadimenol, triazoxide, tricyclazole, tridemorph, trifloxystrobin, triflumizole, triforine, triticonazole, validamycin, vinclozolin, zineb, ziram, zoxamide, Candida oleophila, Fusarium oxysporum, Gliocladium spp., Phlebiopsis gigantean, Streptomyces griseoviridis, Trichoderma spp., (RS)--N-(3,5-dichlorophenyl)-2-(methoxymethyl)-succinimide, 1,2-dichloropropane, 1,3-dichloro-1,1,3,3-tetrafluoroacetone hydrate, 1-chloro-2,4-dinitronaphthalene, 1-chloro-2-nitropropane, 2-(2-heptadecyl-2-imidazolin-1-yl)ethanol, 2,3-dihydro-5-phenyl-1,4-dithi-ine 1,1,4,4-tetraoxide, 2-methoxyethylmercury acetate, 2-methoxyethylmercury chloride, 2-methoxyethylmercury silicate, 3-(4-chlorophenyl)-5-methylrhodanine, 4-(2-nitroprop-1-enyl)phenyl thiocyanateme: ampropylfos, anilazine, azithiram, barium polysulfide, Bayer 32394, benodanil, benquinox, bentaluron, benzamacril; benzamacril-isobutyl, benzamorf, binapacryl, bis(methylmercury) sulfate, bis(tributyltin) oxide, buthiobate, cadmium calcium copper zinc chromate sulfate, carbamorph, CECA, chlobenthiazone, chloraniformethan, chlorfenazole, chlorquinox, climbazole, copper bis(3-phenylsalicylate), copper zinc chromate, cufraneb, cupric hydrazinium sulfate, cuprobam, cyclafuramid, cypendazole, cyprofuram,

decafentin, dichlone, dichlozoline, diclobutrazol, dimethirimol, dinocton, dinosulfon, dinoterbon, dipyrithione, ditalimfos, dodicin, drazoxolon, EBP, ESBP, etaconazole, etem, ethirim, fenaminosulf, fenapanil, fenitropan, fluotrimazole, furcarbanil, furconazole, furconazole-cis, furmecyclox, furophanate, glyodine, griseofulvin, halacrinate, Hercules 3944, hexylthiofos, ICIA0858, isopamphos, isovaledione, mebenil, mecarbinzid, metazoxolon, methfuroxam, methylmercury dicyandiamide, metsulfovax, milneb, mucochloric anhydride, myclozolin, N-3,5-dichlorophenyl-succinimide, N-3-nitrophenylitaconimide, natamycin, N-ethylmercurio-4-toluenesulfonanilide, nickel bis(dimethyldithiocarbamate), OCH, phenylmercury dimethyldithiocarbamate, phenylmercury nitrate, phosdiphen, prothiocarb; prothiocarb hydrochloride, pyracarbolid, pyridinitril, pyroxychlor, pyroxyfur, quinacetol; quinacetol sulfate, quinazamid, quinconazole, rabenzazole, salicylanilide, SSF-109, sultropen, tecoram, thiadifluor, thicyofen, thiochlorfenphim, thiophanate, thioquinox, tioxymid, triamiphos, triarimol, triazbutil, trichlamide, urbacid, XRD-563, and zarilamid, and any combinations thereof.

Some of the herbicides that can be employed in conjunction with the compounds disclosed herein include: amide herbicides such as allidochlor, beflubutamid, benzadox, benzipram, bromobutide, cafenstrole, CDEA, chlorthiamid, cyprazole, dimethenamid, dimethenamid-P, diphenamid, epronaz, etnipromid, fentrazamide, flupoxam, fomesafen, halosafen, isocarbamid, isoxaben, napropamide, naptalam, pethoxamid, propyzamide, quinonamid and tebutam; anilide herbicides such as chloranocryl, cisanilide, clomeprop, cypromid, diflufenican, etobenzanid, fenasulam, flufenacet, flufenican, mefenacet, mefluidide, metamifop, monalide, naproanilide, pentanochlor, picolinafen and propanil; arylalanine herbicides such as benzoylprop, flamprop and flamprop-M; chloroacetanilide herbicides such as acetochlor, alachlor, butachlor, butenachlor, delachlor, diethatyl, dimethachlor, metazachlor, metolachlor, S-metolachlor, pretilachlor, propachlor, propisochlor, prynachlor, terbuchlor, thenylchlor and xylachlor; sulfonanilide herbicides such as benzofluor, perfluidone, pyrimisulfan and profluazol; sulfonamide herbicides such as asulam, carbasulam, fenasulam and oryzalin; antibiotic herbicides such as bilanafos; benzoic acid herbicides such as chloramben, dicamba, 2,3,6-TBA and tricamba; pyrimidinyloxybenzoic acid herbicides such as bispyribac and pyriminobac; pyrimidinylthiobenzoic acid herbicides such as pyrithiobac; phthalic acid herbicides such as chlorthal; picolinic acid herbicides such as aminopyralid, clopyralid and picloram; quinolinecarboxylic acid herbicides such as quinclorac and quinmerac; arsenical herbicides such as cacodylic acid, CMA, DSMA, hexaflurate, MAA, MAMA, MSMA, potassium arsenite and sodium arsenite; benzoylcyclohexanedione herbicides such as mesotrione, sulcotrione, tefuryltrione and tembotrione; benzofuranyl alkylsulfonate herbicides such as benfuresate and ethofumesate; carbamate herbicides such as asulam, carboxazole chlorprocarb, dichlormate, fenasulam, karbutilate and terbucarb; carbanilate herbicides such as barban, BCPC, carbasulam, carbetamide, CEPC, chlorbufam, chlorpropham, CPPC, desmedipham, phenisopham, phenmedipham, phenmedipham-ethyl, propham and swep; cyclohexene oxime herbicides such as alloxydim, butroxydim, clethodim, cloproxydim, cycloxydim, profoxydim, sethoxydim, tepraloxydim and tralkoxydim; cyclopropylisoxazole herbicides such as isoxachlortole and isoxaflutole; dicarboximide herbicides such as benzfendizone, cinidon-ethyl, flumezin, flumiclorac, flumioxazin and flumipropyn; dinitroaniline herbicides such as benfluralin, butralin, dinitramine, ethalfluralin, fluchloralin, isopropalin, methalpropalin, nitralin, oryzalin, pendimethalin, prodiamine, profluralin and trifluralin; din itrophenol herbicides such as dinofenate, dinoprop, dinosam, dinoseb, dinoterb, DNOC, etinofen and medinoterb; diplhenyl ether herbicides such as ethoxyfen; nitrophenyl ether herbicides such as acifluorfen, aclonifen, bifenox, chlomethoxyfen, chlornitrofen, etnipromid, fluorodifen, fluoroglycofen, fluoronitrofen, fomesafen, furyloxyfen, halosafen, lactofen,nitrofen, nitrofluorfen and oxyfluorfen; dithiocarbamate herbicides such as dazomet and metam; halogenated aliphatic herbicides such as alorac, chloropon, dalapon, flupropanate, hexachloroacetone, iodomethane, methyl bromide, monochloroacetic acid, SMA and TCA; imidazolinone herbicides such as imazamethabenz, imazamox, imazapic, imazapyr, imazaquin and imazethapyr; inorganic herbicides such as ammonium sulfamate, borax, calcium chlorate, copper sulfate, ferrous sulfate, potassium azide, potassium cyanate, sodium azide, sodium chlorate and sulfuric acid; nitrile herbicides such as bromobonil, bromoxynil, chloroxynil, dichlobenil, iodobonil, ioxynil and pyraclonil; organophosphorus herbicides such as amiprofos-methyl, anilofos, bensulide, bilanafos, butamifos, 2,4-DEP, DMPA, EBEP, fosamine, glufosinate, glyphosate and piperophos; phenoxy herbicides such as bromofenoxim, clomeprop, 2,4-DEB, 2,4-DEP, difenopenten, disul, erbon, etnipromid, fenteracol and trifopsime; phenoxyacetic herbicides such as 4-CPA, 2,4-D, 3,4-DA, MCPA, MCPA-thioethyl and 2,4,5-T; phenoxybutyric herbicides such as 4-CPB, 2,4-DB, 3,4-DB, MCPB and 2,4,5-TB; phenoxypropionic herbicides such as cloprop, 4-CPP, dichlorprop, dichlorprop-P, 3,4-DP, fenoprop, mecoprop and mecoprop-P; aryloxyphenoxypropionic herbicides such as chlorazifop, clodinafop, clofop, cyhalofop, diclofop, fenoxaprop, fenoxaprop-P, fenthiaprop, fluazifop, fluazifop-P, haloxyfop, haloxyfop-P, isoxapyrifop, metamifop, propaquizafop, quizalofop, quizalofop-P and trifop; phenylenediamine herbicides such as dinitramine and prodiamine; pyrazolyl herbicides such as benzofenap, pyrazolynate, pyrasulfotole, pyrazoxyfen, pyroxasulfone and topramezone; pyrazolyiplpietiyl herbicides such as fluazolate and pyraflufen; pyridaziiie herbicides such as credazine, pyridafol and pyridate; pyridazitiotte herbicides such as brompyrazon, chloridazon, dimidazon, flufenpyr, metflurazon, norflurazon, oxapyrazon and pydanon; pyridinie herbicides such as aminopyralid, cliodinate, clopyralid, dithiopyr, fluroxypyr, haloxydine, picloram, picolinafen, pyriclor, thiazopyr and triclopyr; pyrimidinediamitie herbicides such as iprymidam and tioclorim; quaternary ammonium herbicides such as cyperquat, diethamquat, difenzoquat, diquat, morfamquat and paraquat; thiocarbamate herbicides such as butylate, cycloate, diallate, EPTC, esprocarb, ethiolate, isopolinate, methiobencarb, molinate, orbencarb, pebulate, prosulfocarb, pyributicarb,

sulfallate, thiobencarb, tiocarbazil, tri-allate and vernolate; thiocarbonate herbicides such as dimexano, EXD and proxan; thiourea herbicides such as methiuron; triazine herbicides such as dipropetryn, triaziflam and trihydroxytriazine; chlorotriazine herbicides such as atrazine, chlorazine, cyanazine, cyprazine, eglinazine, ipazine, mesoprazine, procyazine, proglinazine, propazine, sebuthylazine, simazine, terbuthylazine and trietazine; methoxytriazine herbicides such as atraton, methometon, prometon, secbumeton, simeton and terbumeton; methylthiotriazine herbicides such as ametryn, aziprotryne, cyanatryn, desmetryn, dimethametryn, methoprotryne, prometryn, simetryn and terbutryn; triazinone herbicides such as ametridione, amibuzin, hexazinone, isomethiozin, metamitron and metribuzin; triazole herbicides such as amitrole, cafenstrole, epronaz and flupoxam; triazolonc herbicides such as amicarbazone, bencarbazone, carfentrazone, flucarbazone, propoxycarbazone, sulfentrazone and thiencarbazone-methyl; triazolopyrimidine herbicides such as cloransulam, diclosulam, florasulam, flumetsulam, metosulam, penoxsulam and pyroxsulam; uracil herbicides such as butafenacil, bromacil, flupropacil, isocil, lenacil and terbacil; 3-phenyluracils; urea herbicides such as benzthiazuron, cumyluron, cycluron, dichloralurea, diflufenzopyr, isonoruron, isouron, methabenzthiazuron, monisouron and noruron; phenylurea herbicides such as anisuron, buturon, chlorbromuron, chloreturon, chlorotoluron, chloroxuron, daimuron, difenoxuron, dimefuron, diuron, fenuron, fluometuron, fluothiuron, isoproturon, linuron, methiuron, methyldymron, metobenzuron, metobromuron, metoxuron, monolinuron, monuron, neburon, parafluron, phenobenzuron, siduron, tetrafluron and thidiazuron; pyrimidinylsulfonylurea herbicides such as amidosulfuron, azimsulfuron, bensulfuron, chlorimuron, cyclosulfamuron, ethoxysulfuron, flazasulfuron, flucetosulfuron, flupyrsulfuron, foramsulfuron, halosulfuron, imazosulfuron, mesosulfuron, nicosulfuron, orthosulfamuron, oxasulfuron, primisulfuron, pyrazosulfuron, rimsulfuron, sulfometuron, sulfosulfuron and trifloxysulfuron; triazinylsulfonylurea herbicides such as chlorsulfuron, cinosulfuron, ethametsulfuron, iodosulfuron, metsulfuron, prosulfuron, thifensulfuron, triasulfuron, tribenuron, triflusulfuron and tritosulfuron; thiadiazolylurea herbicides such as buthiuron, ethidimuron, tebuthiuron, thiazafluron and thidiazuron; and unclassified herbicides such as acrolein, allyl alcohol, azafenidin, benazolin, bentazone, benzobicyclon, buthidazole, calcium cyanamide, cambendichlor, chlorfenac, chlorfenprop, chlorflurazole, chlorflurenol, cinmethylin, clomazone, CPMF, cresol, ortho-dichlorobenzene, dimepiperate, endothal, fluoromidine, fluridone, flurochloridone, flurtamone, fluthiacet, indanofan, methazole, methyl isothiocyanate, nipyraclofen, OCH, oxadiargyl, oxadiazon, oxaziclomefone, pentachlorophenol, pentoxazone, phenylmercury acetate, pinoxaden, prosulfalin, pyribenzoxim, pyriftalid, quinoclamine, rhodethanil, sulglycapin, thidiazimin, tridiphane, trimeturon, tripropindan and tritac.

It should be understood that while the use of words such as preferable, preferably, preferred or more preferred utilized in the description above indicate that the feature so described may be more desirable, it nonetheless may not be necessary and embodiments lacking the same may be contemplated as within the scope of the invention, the scope being defined by the claims that follow. In reading the claims, it is intended that when words such as "a," "an," "at least one," or "at least one portion" are used there is no intention to limit the claim to only one item unless specifically stated to the contrary in the claim. When the language "at least a portion" and/or "a portion" is used the item can include a portion and/or the entire item unless specifically stated to the contrary.

## Claims

1. A compound according to formula (I)

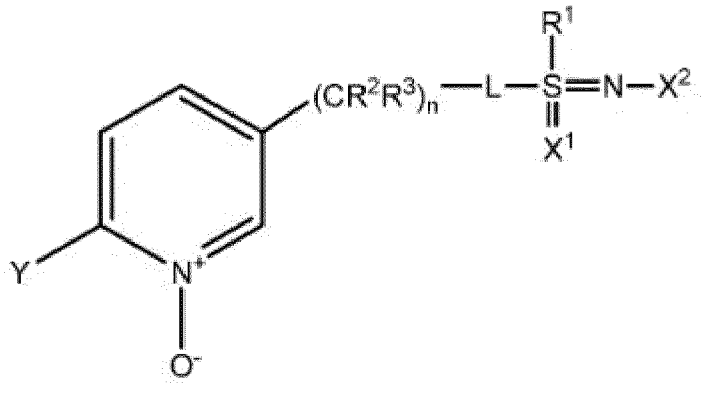

(I)

wherein

L represents a single bond or $R^1$, S and L taken together represent a 4-, 5- or 6-membered ring;
$R^1$ represents ($C_1$-$C_4$) alkyl;
$R^2$ and $R^3$ individually represent hydrogen, methyl, ethyl, fluoro, chloro or bromo;
n is an integer from 0-3;
Y represents ($C_1$-$C_4$) haloalkyl, F, Cl, Br, or I;
$X^1$ is optional and represents O when present;
$X^2$ represents CN; and
$R^4$ represents ($C_1$-$C_3$) alkyl.

**2.** The compound of claim 1, wherein $X^1$ is present.

**3.** The compound of claim 1, wherein Y represents $CF_3$ or Cl.

**4.** The compound of claim 1, wherein $R^2$ and $R^3$ independently represent hydrogen, methyl or ethyl.

**5.** The compound of claim 1, wherein $X^1$, $X^2$, $R^2$, $R^3$, n, and Y are as previously defined, $R^1$ represents $CH_3$, L represents a single bond and the compound of formula (I) has the structure

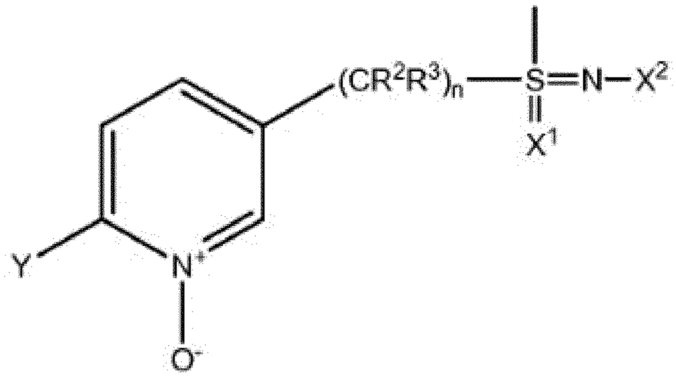

wherein n = 1-3

**6.** The compound of claim 5, wherein $X^1$ is present and represents O, $X^2$ represents CN, Y represents ($C_1$-$C_4$) haloalkyl, $R^2$ and $R^3$ individually represent hydrogen, methyl, ethyl, fluoro, chloro or bromo, and n is an integer from 1-3.

**7.** The compound of claim 6, wherein Y represents $CF_3$, $R^2$ and $R^3$ individually represent hydrogen, methyl or ethyl, and n is an integer from 1-3.

**8.** The compound of claim 1, wherein $X^1$, $X^2$, and Y are as previously defined, n is 0, $R^1$, S and L taken together form a saturated 5-membered ring, and the compound of formula (I) has the structure

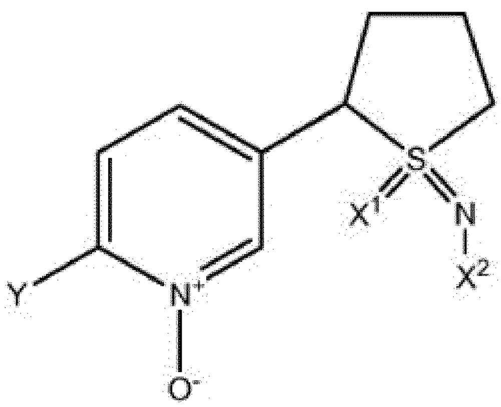

**9.** A compound selected from one of the following:

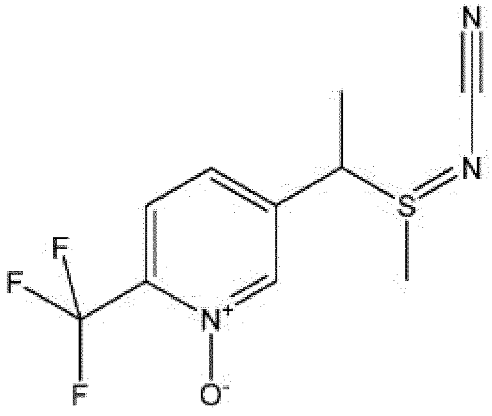

(5)

,

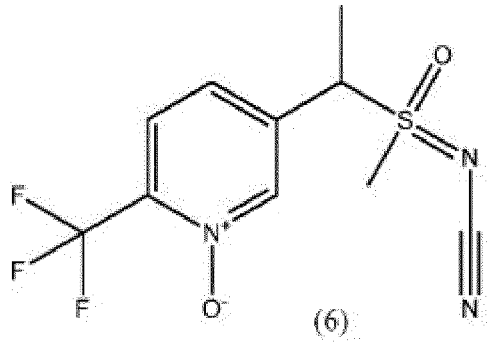

(6)

,

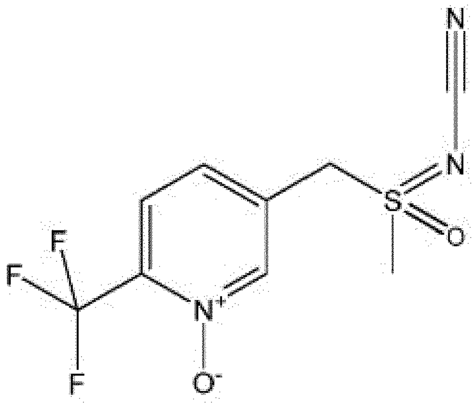

(9)

,

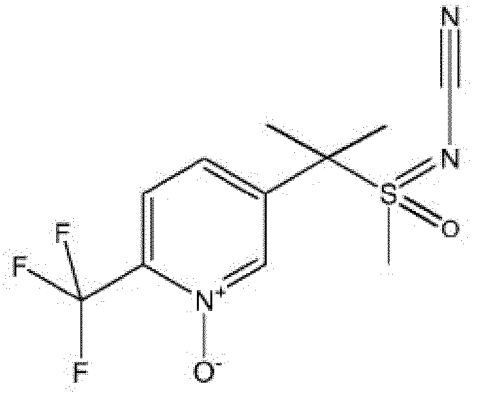

(10)

,

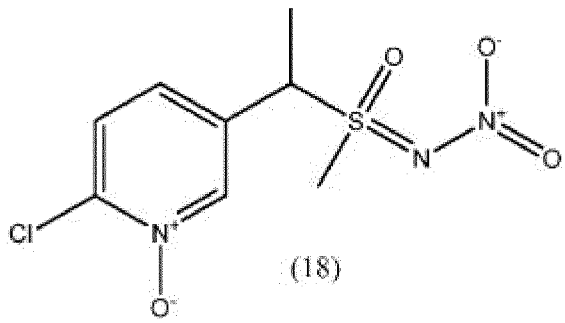

(18)

,

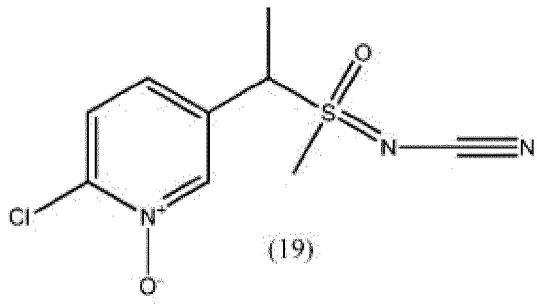

(19)

,

and

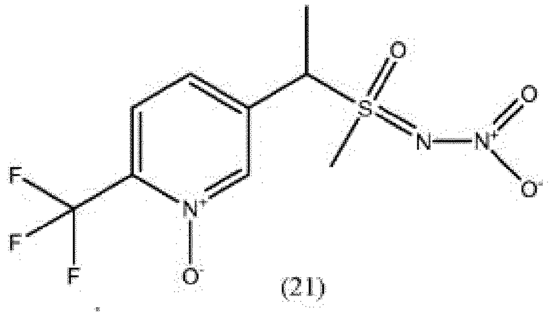

(21)

10. A composition for controlling insects which comprises a compound according to any one of claims 1 and 9 in combination with a phytologically-acceptable carrier.

11. A method of controlling insects which comprises applying to a locus where control is desired an insect-inactivating amount of a compound according to any one of claims 1 and 9, with the proviso that said locus is not the human or animal body.

**Patentansprüche**

1. Eine Verbindung gemäß Formel (I)

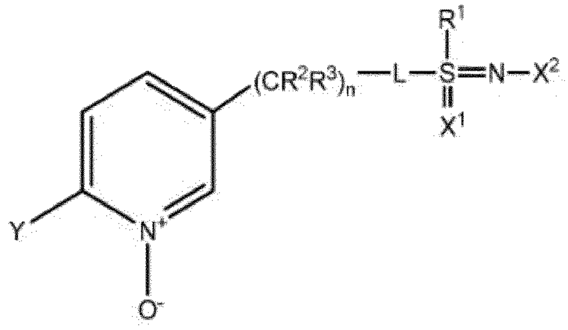

(I)

wobei

L eine Einfachbindung darstellt oder $R^1$, S und L zusammengenommen einen 4-, 5- oder 6-gliedrigen Ring darstellen;
$R^1$ $(C_1-C_4)$-Alkyl darstellt;
$R^2$ und $R^3$ individuell Wasserstoff, Methyl, Ethyl, Fluor, Chlor oder Brom darstellen;
n eine ganze Zahl von 0-3 ist;
Y $(C_1-C_4)$-Halogenalkyl, F, Cl, Br oder I darstellt;
$X^1$ optional ist und O darstellt, sofern vorhanden;
$X^2$ CN darstellt; und
$R^4$ $(C_1-C_3)$-Alkyl darstellt.

2. Die Verbindung gemäß Anspruch 1, wobei $X^1$ vorhanden ist.

3. Die Verbindung gemäß Anspruch 1, wobei Y $CF_3$ oder Cl darstellt.

4. Die Verbindung gemäß Anspruch 1, wobei $R^2$ und $R^3$ unabhängig Wasserstoff, Methyl oder Ethyl darstellen.

5. Die Verbindung gemäß Anspruch 1, wobei $X^1$, $X^2$, $R^2$, $R^3$, n und Y wie zuvor definiert sind, $R^1$ $CH_3$ darstellt, L eine Einfachbindung darstellt und die Verbindung der Formel (I) die Struktur

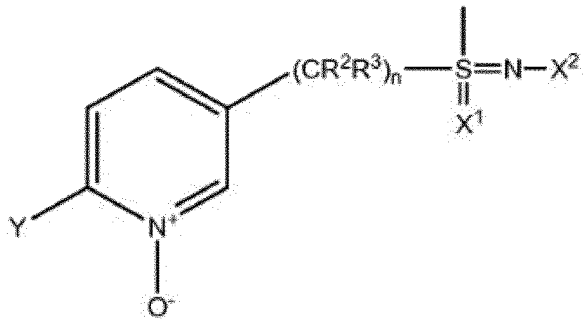

aufweist, wobei n = 1-3 ist.

6. Die Verbindung gemäß Anspruch 5, wobei $X^1$ vorhanden ist und O darstellt, $X^2$ CN darstellt, Y ($C_1$-$C_4$)-Halogenalkyl darstellt, $R^2$ und $R^3$ individuell Wasserstoff, Methyl, Ethyl, Fluor, Chlor oder Brom darstellen und n eine ganze Zahl von 1-3 ist.

7. Die Verbindung gemäß Anspruch 6, wobei Y $CF_3$ darstellt, $R^2$ und $R^3$ individuell Wasserstoff, Methyl oder Ethyl darstellen und n eine ganze Zahl von 1-3 ist.

8. Die Verbindung gemäß Anspruch 1, wobei $X^1$, $X^2$ und Y wie zuvor definiert sind, n 0 ist, $R^1$, S und L zusammengenommen einen gesättigten 5-gliedrigen Ring bilden und die Verbindung der Formel (I) die Struktur

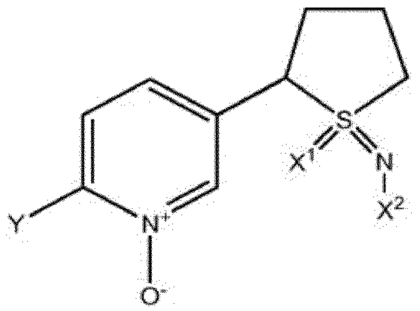

.

aufweist.

9. Eine Verbindung ausgewählt aus einer der folgenden:

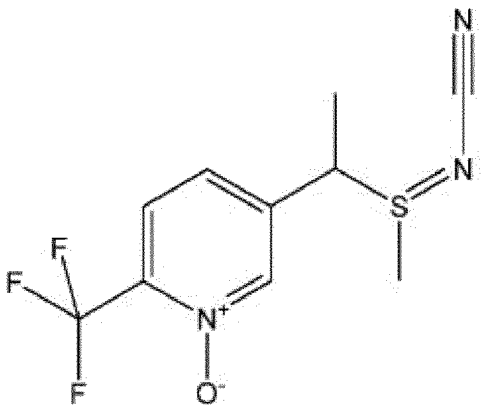

(5)

,

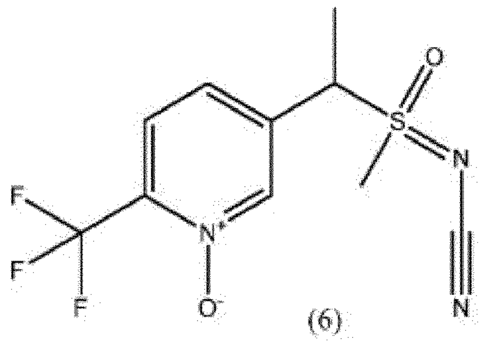

(6)

,

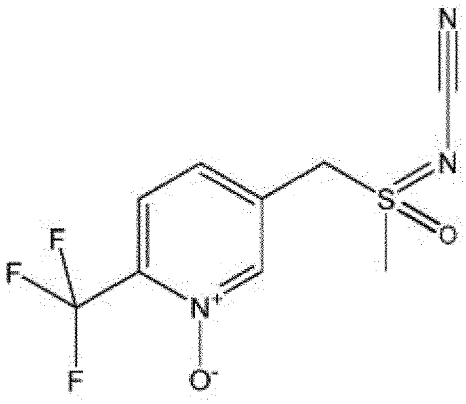

(9)

,

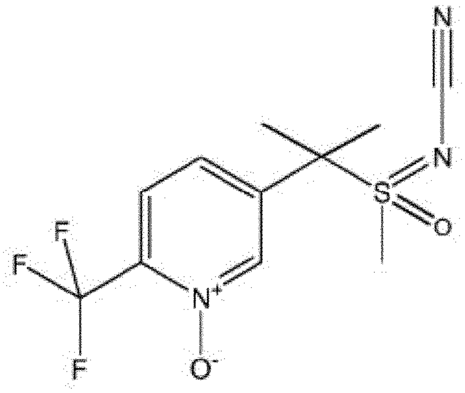

(10)

,

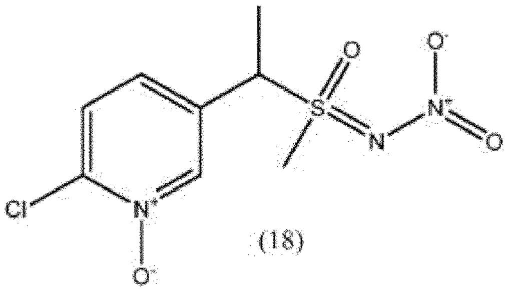

(18)

,

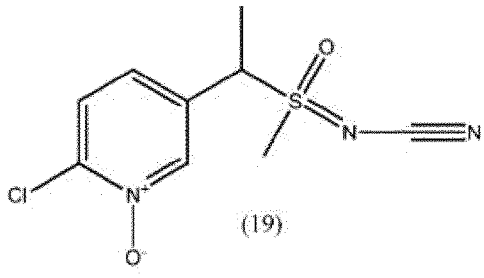

(19)

,

und

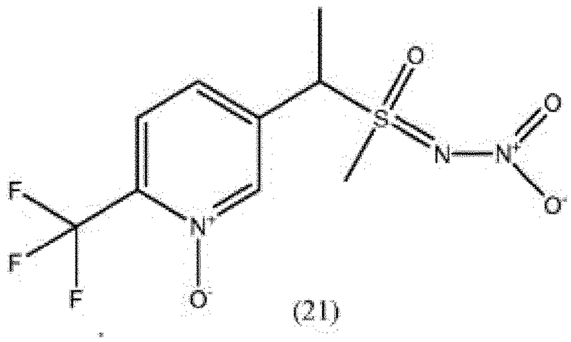

(21)

.

**10.** Eine Zusammensetzung zur Bekämpfung von Insekten, welche eine Verbindung gemäß irgendeinem der Ansprüche 1 und 9 in Kombination mit einem phytologisch akzeptablen Trägerstoff umfasst.

**11.** Ein Verfahren zur Bekämpfung von Insekten, welche das Anwenden einer Insekten inaktivierenden Menge einer Verbindung gemäß irgendeinem der Ansprüche 1 und 9 auf einen Ort, an welchem Bekämpfung erwünscht ist, umfasst, unter der Voraussetzung, dass dieser Ort nicht der menschliche oder tierische Körper ist.

**Revendications**

**1.** Composé selon la formule (I)

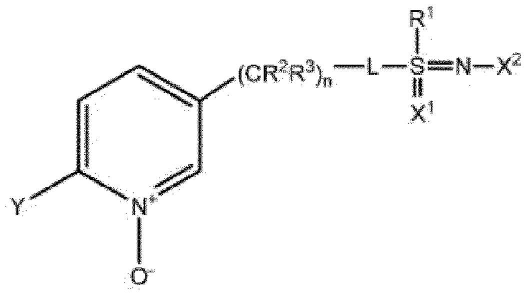

(I)

dans laquelle

L représente une liaison simple ou $R^1$, S et L pris ensemble représentent un cycle à 4, 5 ou 6 chaînons ;
$R^1$ représente un groupe alkyle en $C_1$-$C_4$ ;
$R^2$ et $R^3$ représentent individuellement un atome d'hydrogène, un groupe méthyle, éthyle, fluoro, chloro ou bromo ;
n est un nombre entier valant de 0 à 3 ;
Y représente un groupe halogénoalkyle($C_1$-$C_4$), un atome de fluor, de chlore, de brome ou d'iode ;
$X^1$ est en option et, lorsqu'il est présent, représente un atome d'oxygène ;
$X^2$ représente un groupe CN ; et
$R^4$ représente un groupe alkyle en $C_1$-$C_3$.

**2.** Composé selon la revendication 1, dans lequel $X^1$ est présent.

**3.** Composé selon la revendication 1, dans lequel Y représente un groupe $CF_3$ ou un atome de chlore.

**4.** Composé selon la revendication 1, dans lequel $R^2$ et $R^3$ représentent indépendamment un atome d'hydrogène, un groupe méthyle ou éthyle.

**5.** Composé selon la revendication 1, dans lequel $X^1$, $X^2$, $R^2$, $R^3$, n et Y sont tels que définis précédemment, $R^1$ représente un groupe $CH_3$, L représente une liaison simple et le composé de formule (I) présente la structure

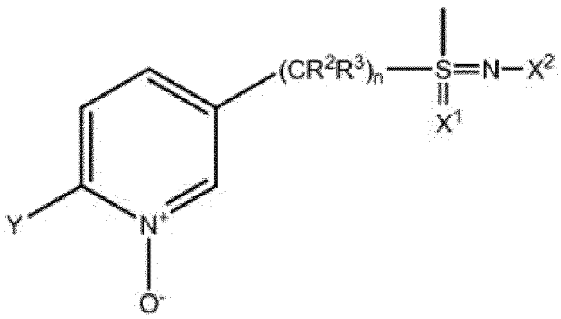

dans laquelle n = 1-3.

**6.** Composé selon la revendication 5, dans lequel $X^1$ est présent et représente un atome d'oxygène, $X^2$ représente un groupe CN, Y représente un groupe halogénoalkyle($C_1$-$C_4$), $R^2$ et $R^3$ représentent individuellement un atome d'hydrogène, un groupe méthyle, éthyle, fluoro, chloro ou bromo, et n est un nombre entier valant de 1 à 3.

**7.** Composé selon la revendication 6, dans lequel Y représente un groupe $CF_3$, $R^2$ et $R^3$ représentent individuellement un atome d'hydrogène, un groupe méthyle ou éthyle, et n est un nombre entier valant de 1 à 3.

**8.** Composé selon la revendication 1, dans lequel $X^1$, $X^2$, et Y sont tels que définis précédemment, n est 0, $R^1$, S et L pris ensemble forment un cycle saturé à 5 chaînons, et le composé de formule (I) présente la structure

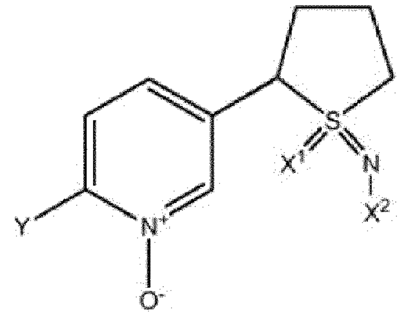

.

**9.** Composé choisi parmi l'un des suivants :

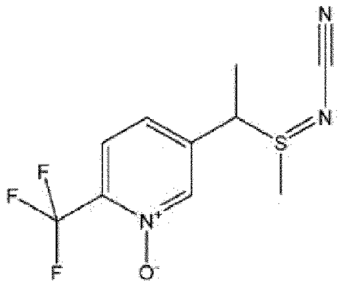

(5) ,

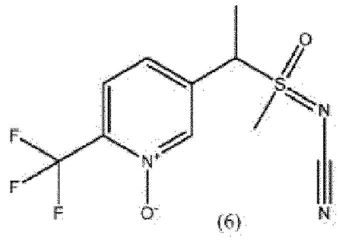

(6) ,

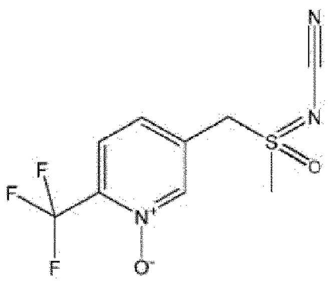

(9) ,

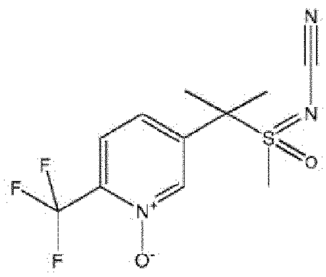

(10) ,

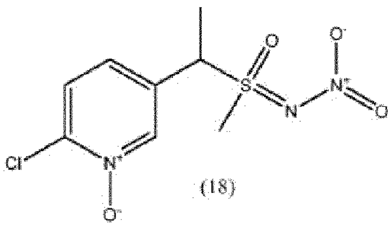

(18) ,

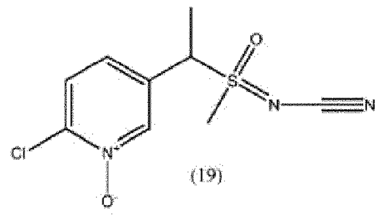

(19) ,

et

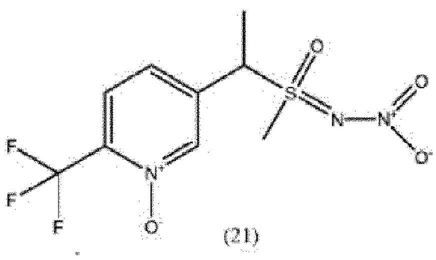

(21) .

**10.** Composition destinée à la lutte contre des insectes, qui comprend un composé selon l'une quelconque des revendications 1 et 9 en association avec un véhicule phytologiquement acceptable.

**11.** Procédé de lutte contre des insectes, qui comprend le fait d'appliquer sur un lieu où la lutte est désirée une quantité inactivant des insectes d'un composé selon l'une quelconque des revendications 1 et 9, étant entendu que ledit lieu n'est pas le corps humain ou animal.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7678920 B
- US 7687634 B
- US 8188292 B
- CN 102070607
- US 5302605 A
- US 20080033180 A
- WO 2010002577 A


### Non-patent literature cited in the description


- **W.S. ABBOTT.** A Method of Computing the Effectiveness of an Insecticide. *J. Econ. Entomol.,* 1925, vol. 18, 265-267